# FASCICULE DE BREVET EUROPEEN

(11) **EP 3 189 334 B1**
(45) Date de publication et mention de la délivrance du brevet: **16.04.2025**
(21) Numéro de dépôt: 15756684.5
(22) Date de dépôt: 11.08.2015
(51) Int. Cl.: G01N 33/68, G01N 33/569, C12Q 1/06

(54) **PROCÉDÉ DE QUANTIFICATION D'AU MOINS UN GROUPE DE MICROORGANISMES PAR SPECTROMÉTRIE DE MASSE**
VERFAHREN ZUR QUANTIFIZIERUNG VON MINDESTENS EINER MIKROORGANISMUSGRUPPE ÜBER MASSENSPEKTROMETRIE
METHOD FOR QUANTIFYING AT LEAST ONE MICROORGANISM GROUP VIA MASS SPECTROMETRY

(30) Priorité: 14.08.2014 FR 1457828
(43) Date de publication de la demande: 12.07.2017
(73) Titulaire: Biomérieux, 69280 Marcy l'Étoile (FR)
(72) Inventeur: BARDET, Chloé, F-69007 Lyon (FR); CECCHINI, Tiphaine, F-69290 Saint Genis Les Ollières (FR); CHARRETIER, Yannick, F-69690 Courzieu (FR); CHARRIER, Jean-Philippe, F-69160 Tassin La Demi-lune (FR); COMPAGNON, Christelle, F-69007 Lyon (FR); FORTIN, Tanguy, F-69006 Lyon (FR)
(74) Mandataire: bioMérieux PI Groupement mandataires
(86) Numéro de dépôt international: PCT/FR2015/052196
(87) Numéro de publication internationale: WO 2016/024068

(56) Documents cités:
- EP-A1- 2 124 060
- WO-A1-2012/092302
- WO-A2-01/70955
- WO-A2-02/077183
- WO-A2-2006/079076
- US-A1- 2004 259 226
- US-A1- 2007 178 450
- US-B1- 6 551 795
- SHENG PAN ET AL: "Mass Spectrometry Based Targeted Protein Quantification: Methods and Applications", JOURNAL OF PROTEOME RESEARCH, vol. 8, no. 2, 6 February 2009 (2009-02-06), pages 787 - 797, XP055191740, ISSN: 1535-3893, DOI: 10.1021/pr800538n
- T. C. WALTHER ET AL: "Mass spectrometry-based proteomics in cell biology", SCIENCE, vol. 327, no. 5968, 23 August 2010 (2010-08-23), pages 1000 - 500, XP055179058, ISSN: 0036-8075, DOI: 10.1126/science.1179689
- ROSS P L ET AL: "Multiplexed protein quantitation in saccharomyces cerevisiae using amine-reactive isobaric tagging reagents", MOLECULAR & CELLULAR PROTEOMICS, AMERICAN SOCIETY FOR BIOCHEMISTRY AND MOLECULAR BIOLOGY, vol. 3, no. 12, 1 December 2004 (2004-12-01), pages 1154 - 1169, XP002328690, ISSN: 1535-9476, DOI: 10.1074/MCP.M400129-MCP200
- DATABASE UniParc [online] 28 July 1992 (1992-07-28), XP002751647, Database accession no. UPI000016F527
- LANGE VINZENZ ET AL: "Targeted quantitative analysis of Streptococcus pyogenes virulence factors by multiple reaction monitoring", MOLECULAR & CELLULAR PROTEOMICS, AMERICAN SOCIETY FOR BIOCHEMISTRY AND MOLECULAR BIOLOGY, vol. 7, no. 8, 1 August 2008 (2008-08-01), pages 1489 - 1500, XP009107637, ISSN: 1535-9476, DOI: 10.1074/MCP.M800032-MCP200

## Description

La présente invention concerne le domaine de la bactériologie. Plus précisément, l'invention concerne la quantification de microorganismes issus d'un échantillon en utilisant la spectrométrie de masse.

Depuis la découverte des microbes par Pasteur, les microorganismes sont étudiés par microscopie et analyses biochimiques et quantifiés par cultures quantitatives. Ces méthodes traditionnelles sont souvent longues et fastidieuses et des alternatives analytiques ont très tôt été recherchées. C'est ainsi que l'analyse de bactéries par spectrométrie de masse a été initiée dès 1975 par J. Anhalt et C. Fenselau [1]

Ces travaux préliminaires ont été suivis par l'étude en chromatographie en phase gazeuse couplée à la spectrométrie de masse (GC-MS) d'acides gras de la paroi des microorganismes [2]. Cette méthode a été popularisée sous l'appellation anglo-saxonne de FAME pour Fatty Acid Methyl Ester. Elle constitue actuellement une méthode de référence pour les études taxonomiques. Son utilisation reste cependant limitée à certains laboratoires spécialisés maîtrisant le traitement de l'échantillon par saponification, hydrolyse et dérivation.

En 1996, les travaux de M. Claydon et al [3] ainsi que de T. Krishnamurthy et P. Ross [4] ont montré la possibilité d'identifier différentes espèces bactériennes avec un spectromètre de masse de type MALDI-TOF (acronyme de l'anglais Matrix Assisted Laser Desorption Ionization - Time Of Flight). L'analyse associe l'acquisition d'un spectre de masse et l'interprétation d'un logiciel expert. Elle est extrêmement simple et peut être effectuée en quelques minutes. Cependant elle ne se diffuse que depuis très peu de temps dans les laboratoires d'analyses médicales [5]. Son utilisation clinique est actuellement limitée à l'identification d'espèces bactériennes et de levures. Elle n'est pas utilisée pour la quantification.

Or la quantification des microorganismes est fondamentale tant dans le domaine clinique que le domaine industriel. Ainsi, par exemple, la quantification de bactéries est un élément essentiel dans le diagnostic de certaines infections, afin d'assurer une prise en charge optimale des patients. De même, la quantification de bactéries est importante dans l'étude de la quantité de certaines protéines exprimées par la bactérie.

De nombreuses applications ont été développées dans la quantification absolue ou relative de protéines microbiennes par spectrométrie de masse MS/MS, mais aucune application dans la quantification de microorganismes par spectrométrie de masse MS/MS. [6]

Le document US2004/0259226 décrit des méthodes de quantification de microorganismes impliqués dans le bioterrorisme par mesure d'un marqueur microbien, qu'est la protéine chaperon 60 (cpn60). Ce document décrit différentes méthodes de quantification et cite parmi ces méthodes, la spectrométrie de masse et en particulier la spectrométrie de masse de type MALDI-TOF comme étant une technique possible pour quantifier des protéines ou des peptides. Ce document se contente juste de citer cette technique, sans prouver sa pertinence et son efficacité par le biais d'exemples concrets. On sait par ailleurs aujourd'hui que la proteine chaperon 60 a une masse élevée (voisine de 60 kDa) qui rend sa détection très difficile par MALDI-TOF dans un échantillon complexe, tel qu'un microorganisme. En effet, lors d'une ionisation MALDI, les différentes molécules qui composent l'échantillon rentre en compétition et seules les molécules les plus abondantes et de plus faibles masses sont facilement ionisées et détectées. Ainsi l'identification de microorganismes par MALDI-TOF utilise classiquement une détection entre 2 et 20 kDa, ce qui est beaucoup plus faible que la masse de la protéine chaperon 60. De plus, la reproductibilité de l'ionisation MALDI est médiocre. L'ionisation dépend beaucoup de la matrice utilisée, de la reproductibilité du dépôt, c'est à dire de la qualité du mélange de l'échantillon et de la matrice, mais également de la localisation et du nombre de tirs de laser. De ce fait, la quantification de protéines ou peptides est inadaptée au dosage de la protéine chaperon 60.

Les méthodes standards pour l'analyse quantitative de microorganismes sont, la méthode de dénombrement sur boite et la méthode d'analyse de la turbidité par spectrophotométrie.

La méthode de dénombrement sur boite consiste en des dilutions en série d'un échantillon dans du sérum physiologique ensemencées sur des boites de Petri, jusqu'à l'obtention d'une boite sur laquelle les colonies sont dénombrables de manière précise.

L'analyse par spectrophotométrie consiste à analyser la densité optique d'un échantillon, qui sert d'indicateur de croissance de la bactérie, mais ne permet pas une quantification absolue de tous les groupes bactériens présents [7].

D'autres techniques utilisées dans la quantification de bactéries ont été développées mettant en œuvre des méthodes de biologie moléculaires. (Polymerase Chain Reaction, microarray) [8]. Cependant, la technique de PCR qui peut être utilisée en laboratoire de microbiologie clinique, ne permet pas facilement de multiplexer la quantification d'au moins un groupe bactérien avec un autre ou avec la quantification de protéines pouvant apporter un intérêt clinique quant au phénotype de résistance dudit groupe bactérien.

Plus récemment des techniques de cytométrie en flux ont proposé un procédé pouvant répondre aux besoins cliniques concernant la quantification [9].

Ces techniques sont toutefois difficilement applicables à une utilisation clinique de routine. Elles mettent en œuvre des instruments nécessitant un personnel qualifié. Les temps d'analyse, souvent supérieurs à une heure par échantillon, sont incompatibles avec la charge de travail d'un laboratoire d'analyse microbiologique.

La demande US 2004/259226 A1, décrit une méthode pour détecter la présence, l'absence ou la quantité de microorganismes utilisés en bioterrorisme, par détection de la présence, ou l'absence de la protéine cpn60 dans l'échantillon, utilisée comme biomarqueur. La protéine cpn60 qui est une protéine chaperonne ou encore appelée protéine de choc thermique (« Heat Shock Protein » ou hsp) est une protéine ubiquitaire. Par ailleurs, le gène codant cette protéine présente un bon nombre de régions conservées mais également des régions qui varient d'une espèce à l'autre et qui permettent ainsi de détecter par le biais notamment de techniques de biologie moléculaire, spécifiquement certaines espèces impliquées dans le bioterrorisme. Ainsi, ce document décrit que les techniques PCR ou FISH permettent de détecter mais également quantifier les microorganismes par mesure quantitative de cpn60. C'est le cas notamment de la real time PCR, telle que présenté en exemple 2.

Dans ce contexte, l'objectif de la présente Demande est de proposer un procédé de quantification spécifique de microorganismes, à savoir identification et détermination de la quantification d'au moins un groupe de microorganismes qui permette de palier les inconvénients des procédés de l'art antérieur, à savoir fournir un procédé peu coûteux, sans réactifs spécifiques à chaque espèce, notamment par rapport aux procédés de biologie moléculaire, permettant une analyse sélective de protéines ou peptides, donnant un résultat en un temps court, inférieur à une heure, et utilisable en clinique de routine, sans nécessiter un personnel hautement qualifié. De plus, l'ensemble du procédé de quantification spécifique du groupe bactérien ainsi que des protéines d'intérêt dans la caractérisation dudit groupe bactérien, peut être avantageusement réalisé avec un même spectromètre de masse, ce qui simplifie l'instrumentation du laboratoire d'analyse microbiologique.

A cette fin, l'objet de l'invention concerne un nouveau procédé de quantification d'au moins un groupe de microorganismes par au moins une analyse en spectrométrie de masse comprenant au moins une étape de séparation et de fragmentation, ledit procédé comprenant par ailleurs une étape consistant à mesurer la quantité d'au moins un peptide représentatif dudit groupe de microorganismes, dont la quantité produite ou exprimée ne varie pas en fonction de la phase de croissance dans laquelle se trouve ledit groupe de microorganismes, ni en fonction du type de culture ayant subi ledit groupe de microorganismes, ni en fonction des souches utilisées faisant partie d'un seul et même groupe de microorganismes, ledit au moins un peptide représentatif étant obtenus après l'étape de séparation et de fragmentation et jouant le rôle de marqueur(s) de quantification, la quantité dudit ou desdits marqueur(s) de quantification étant directement corrélable à la quantité du au moins un groupe de microorganismes, ledit procédé étant caractérisé en ce que :
- le groupe de microorganismes est constitué par l'espèce Pseudomonas aeruginosa et ledit peptide est de séquence SEQ ID NO :6, et/ou
- le groupe de microorganismes est constitué par l'espèce Escherichia coli et ledit peptide est de séquence SEQ ID NO :2, et/ou
- le groupe de microorganismes est constitué par l'espèce Staphylococcus aureus et ledit peptide est de séquence SEQ ID NO :4 et, et/ou
- le groupe de microorganismes est constitué par la famille des Entérobactéries et ledit peptide est de séquence SEQ ID NO :8.

Les groupes de microorganismes qui peuvent être quantifiés par le procédé de l'invention sont tous des groupes de microorganismes représentant une famille, un genre ou une espèce de microorganismes, pathogènes ou non.

L'échantillon sur lequel le procédé peut être mis en œuvre est tout échantillon susceptible de contenir un microorganisme cible. L'échantillon peut être d'origine biologique, animale (vétérinaire), végétale ou humaine (clinique). Il peut alors correspondre à un prélèvement de fluide biologique (prélèvement bronchique, sang total, sérum, plasma, urine, liquide céphalo-rachidien, sécrétion organique, par exemple), un prélèvement tissulaire ou des cellules isolées. Ce prélèvement peut être utilisé tel quel dans la mesure où les marqueurs de caractérisation des microorganismes sont disponibles dans l'échantillon testé, ou bien il peut subir préalablement à l'analyse, une préparation de type extraction, concentration, purification, culture, selon des méthodes connues de l'homme du métier.

L'échantillon peut être d'origine industrielle dans le cas d'un contrôle microbiologique. Selon une liste non exhaustive, il peut s'agir d'un prélèvement d'air, d'un prélèvement d'eau, d'un prélèvement effectué sur une surface, une pièce ou un produit manufacturé, un produit d'origine alimentaire. Parmi les échantillons d'origine alimentaire, on peut citer de façon non exhaustive un échantillon de produit lacté (yaourts, fromages, etc...), de viande, de poisson, d'œuf, de fruit, de légume, d'eau, de boisson (lait, jus de fruits, soda, etc...). Ces échantillons d'origine alimentaire peuvent aussi provenir de sauces ou de plats élaborés. Un échantillon alimentaire peut enfin être issu d'une alimentation destinée aux animaux, telle que notamment des farines animales.

Par microorganismes, on entend en particulier les bactéries, les levures, les moisissures ou les virus.

Par marqueurs de l'identité et de la quantité d'au moins un groupe de microorganismes, on entend des molécules, d'origine protéique, qui sont caractéristiques desdites propriétés.

Le procédé peut comporter une étape consistant à réaliser l'identification du au moins un groupe de microorganismes simultanément à la quantification dudit au moins un groupe de microorganismes.

Par identification d'un groupe de microorganismes, on entend la différentiation de plusieurs espèces au sein d'un même genre ou bien d'autres genres de microorganismes, la différentiation de plusieurs genres au sein d'une même famille ou bien de différentes familles de microorganismes, la différentiation de plusieurs familles au sein d'une même classe ou bien de différentes classes de microorganismes.

Par quantification d'au moins un groupe de microorganismes dans un échantillon, on entend la quantification d'un marqueur protéique, peptidique ou représentatif de la quantité du ou des groupe(s) de microorganismes dans un échantillon. Une molécule marqueur de quantification d'au moins un groupe de microorganismes est une molécule dont la quantité produite ou exprimée ne varie pas en fonction de la phase de croissance dans laquelle se trouve ledit groupe de microorganismes, ni en fonction du type de culture (gélose ou bouillon) ayant subi ledit groupe de microorganismes, ni en fonction des souches utilisées faisant partie d'un seul et même groupe de microorganismes.

Un autre objet de l'invention concerne un procédé de mesure du taux d'expression d'au moins un peptide et/ou d'au moins une protéine d'intérêt d'un groupe de microorganismes, par au moins une analyse en spectrométrie de masse comprenant au moins une étape de séparation et de fragmentation, ledit procédé comprenant les étapes suivantes :
a) Mesurer la quantité du au moins un peptide d'intérêt et/ou de la au moins une protéine d'intérêt,
b) mesurer la quantité d'au moins un peptide représentatif dudit groupe de microorganismes, dont la quantité produite ou exprimée ne varie pas en fonction de la phase de croissance dans laquelle se trouve ledit groupe de microorganismes, ni en fonction du type de culture ayant subi ledit groupe de microorganismes, ni en fonction des souches utilisées faisant partie d'un seul et même groupe de microorganismes, jouant le rôle de marqueur(s) de quantification, la quantité dudit ou desdits marqueur(s) de quantification étant directement corrélable à la quantité du au moins un groupe de microorganismes,
c) déduire le taux d'expression du au moins un peptide et/ou de la au moins une protéine d'intérêt dudit groupe de microorganismes,

le au moins un peptide d'intérêt et/ou la au moins une protéine d'intérêt, ainsi que le au moins un peptide représentatif ou la au moins une protéine représentative dudit groupe de microorganismes étant obtenus après la au moins une étape de séparation et de fragmentation,
ledit procédé étant caractérisé en ce que :
   - le groupe de microorganismes est constitué par le genre Enterococcus et ledit peptide représentatif est choisi parmi les peptides de séquences SEQ ID NOs :10, 12, et 14, et/ou
   - le groupe de microorganismes est constitué par l'espèce Klebsiella pneumoniae et ledit peptide représentatif est choisi parmi les peptides de séquences SEQ ID NOs : 16, 18, et 20.

De façon plus précise, l'étape c) du procédé selon un second objet de l'invention consiste à comparer la quantité mesurée du au moins un peptide d'intérêt et/ou de la au moins une protéine d'intérêt à la quantité du au moins un peptide représentatif ou de la au moins une protéine représentative dudit groupe de microorganismes jouant le rôle de marqueur(s) de quantification, de sorte qu'il est ainsi possible de déduire un taux d'expression relatif par rapport à la quantité dudit ou desdits marqueur(s) de quantification.

De façon avantageuse, le procédé selon l'invention comporte une étape complémentaire b₁) consistant à déterminer la quantité du au moins un groupe de microorganismes à partir de la quantité dudit ou desdits marqueur(s) de quantification, obtenue à l'étape b). Cette étape complémentaire est réalisée de façon similaire au procédé selon le premier objet de l'invention.

Il convient de noter que les marqueurs de quantification spécifiques d'un groupe de microorganismes sont également utilisables comme marqueur d'identification dudit groupe de microorganismes.

Les buts et avantages de la présente invention seront mieux compris à la lumière de la description détaillée qui suit, en lien avec le dessin dans lequel :

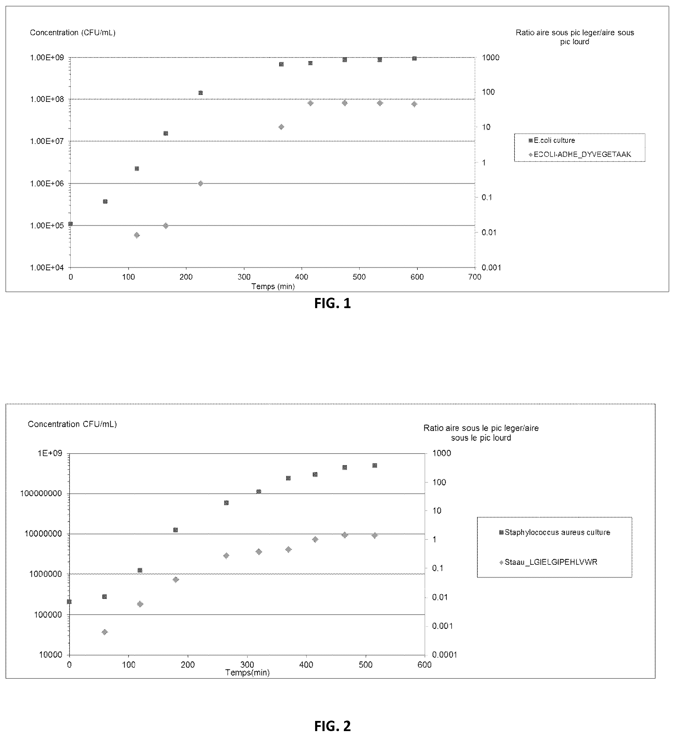

- La figure 1 montre, pour une souche d'*Escherichia coli,* l'évolution en fonction du temps de culture de la quantité de bactéries mesurée par dénombrement sur boites de Petri après dilutions successives (axe de gauche) et par spectrométrie de masse MRM (axe de droite). Dans cet exemple, le signal MRM représentatif de la quantité de bactéries est le signal du peptide de séquence SEQ ID N° 2 spécifique d'*Escherichia coli.* Il est mesuré par le ratio de l'aire sous le pic chromatographique du peptide naturel divisé par l'air sous le pic chromatographique d'un peptide de calibration synthétique, ayant la même séquence que le peptide naturel, mais synthétisé avec de la lysine et de l'arginine alourdis à l'aide d'azote 15 (¹⁵N) et de carbone 13 (¹³C).
- La figure 2 montre, pour une souche d'*Escherichia coli,* l'évolution en fonction du temps de culture de la quantité de bactéries mesurée par dénombrement sur boites de Petri après dilutions successives (axe de gauche) et par spectrométrie de masse MRM (axe de droite). Dans cet exemple, le signal MRM représentatif de la quantité de bactéries est le signal du peptide de séquence SEQ ID N° 4 spécifique de *Staphylococcus aureus.* Il est mesuré par le ratio de l'aire sous le pic chromatographique du peptide naturel divisé par l'air sous le pic chromatographique d'un peptide de calibration synthétique, ayant la même séquence que le peptide naturel, mais synthétisé avec de la lysine et de l'arginine alourdis à l'aide d'azote 15 (¹⁵N) et de carbone 13 (¹³C).

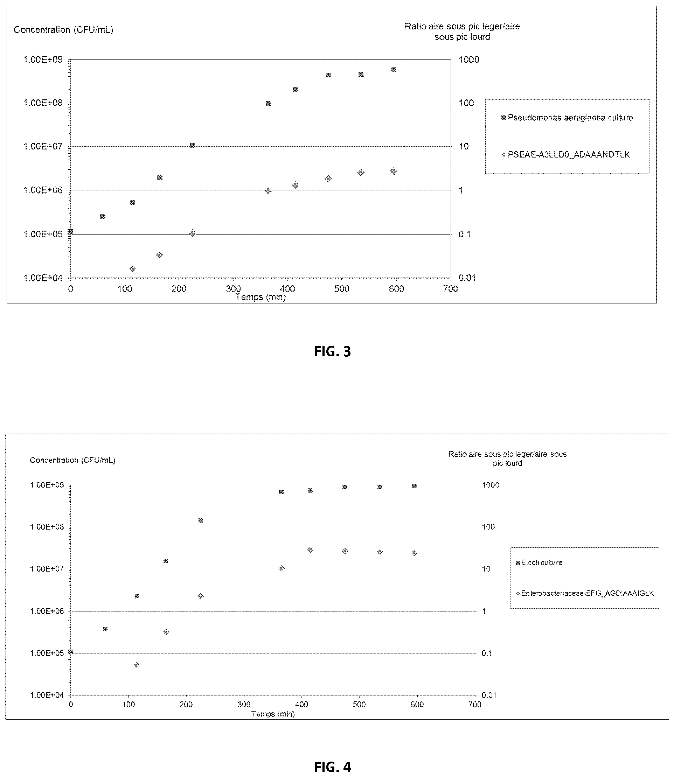

- La figure 3 montre, pour une souche de *Pseudomonas aeruginosa,* l'évolution en fonction du temps de culture de la quantité de bactéries mesurée par dénombrement sur boites de Petri après dilutions successives (axe de gauche) et par spectrométrie de masse MRM (axe de droite). Dans cet exemple, le signal MRM représentatif de la quantité de bactéries est le signal du peptide de séquence SEQ ID N° 6 spécifique de *Pseudomonas aeruginosa.* Il est mesuré par le ratio de l'aire sous le pic chromatographique du peptide naturel divisé par l'air sous le pic chromatographique d'un peptide de calibration synthétique, ayant la même séquence que le peptide naturel, mais synthétisé avec de la lysine et de l'arginine alourdis à l'aide d'azote 15 (¹⁵N) et de carbone 13 (¹³C).
- La figure 4 montre, pour une souche de *Staphylococcus aureus,* l'évolution en fonction du temps de culture de la quantité de bactéries mesurée par dénombrement sur boites de Petri après dilutions successives (axe de gauche) et par spectrométrie de masse MRM (axe de droite). Dans cet exemple, le signal MRM représentatif de la quantité de bactéries est le signal du peptide de séquence SEQ ID N° 8 spécifique de la famille des Entérobactéries. Il est mesuré par le ratio de l'aire sous le pic chromatographique du peptide naturel divisé par l'air sous le pic chromatographique d'un peptide de calibration synthétique, ayant la même séquence que le peptide naturel, mais synthétisé avec de la lysine et de l'arginine alourdis à l'aide d'azote 15 (¹⁵N) et de carbone 13 (¹³C).

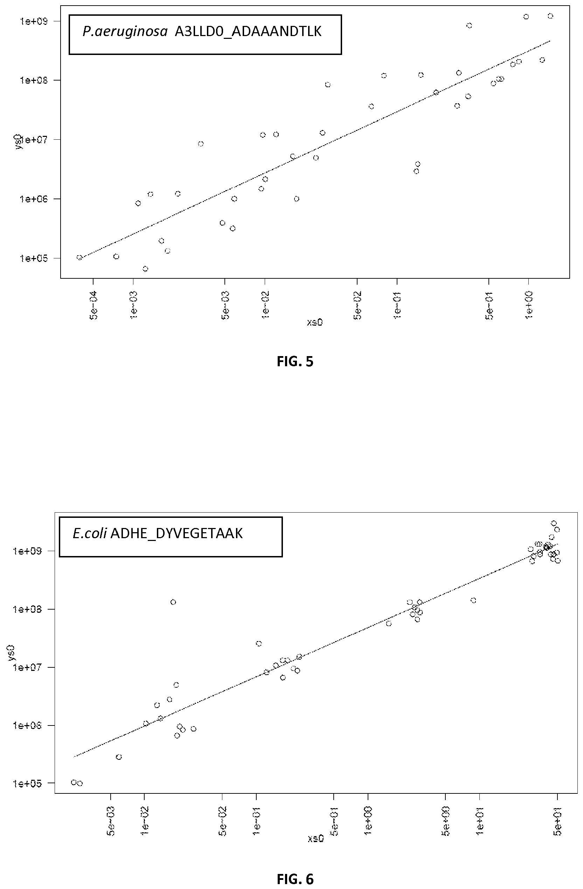

- La figure 5 montre, pour *Pseudomonas aeruginosa,* la corrélation entre la concentration de bactéries présentes dans l'échantillon et la quantité de peptide de séquence SEQ ID N° 6 mesurée par spectrométrie de masse MRM pour plusieurs souches de *Pseudomonas aeruginosa.* La droite de corrélation tracée sur cette figure permet de calculer la quantité de bactéries à partir du signal MRM mesuré comme indiqué figure 3.
- La figure 6 montre, pour *Escherichia coli,* la corrélation entre la concentration de bactéries présentes dans l'échantillon et la quantité de peptide de séquence SEQ ID N° 2 mesurée par spectrométrie de masse ESI-MS/MS pour plusieurs souches de *Escherichia coli.* La droite de corrélation tracée sur cette figure permet de calculer la quantité de bactéries à partir du signal MRM mesuré comme indiqué figure 1.

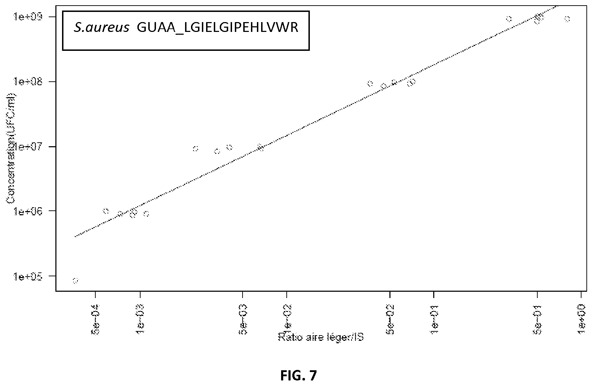

- La figure 7 montre, pour *Staphylococcus aureus,* la corrélation entre la concentration de bactéries présentes dans l'échantillon et la quantité de peptide de séquence SEQ ID N° 4 mesurée par spectrométrie de masse ESI-MS/MS pour plusieurs souches de *Staphylococcus aureus.* La droite de corrélation tracé sur cette figure permet de calculer la quantité de bactéries à partir du signal MRM mesuré comme indiqué figure 2.

Le procédé de l'invention peut être mis en œuvre pour quantifier des espèces bactériennes.

Ainsi, l'espèces bactériennes pouvant être quantifiées selon le procédé de l'invention, on peut citer *Escherichia coli* en utilisant l'alcool déshydrogénase ADHE comme marqueur de quantification ou un peptide appartenant à cette protéine. La séquence de la protéine ADHE est la séquence SEQ ID N° 1 :

SEQ ID N° 1 :
MAVTNVAELNALVERVKKAQREYASFTQEQVDKIFRAAALAAADARIPLAKM
AVAESGMGIVEDKVIKNHFASEYIYNAYKDEKTCGVLSEDDTFGTITIAEPIGIIC
GIVPTTNPTSTAIFKSLISLKTRNAIIFSPHPRAKDATNKAADIVLQAAIAAGAPK
DLIGWIDQPSVELSNALMHHPDINLILATGGPGMVKAAYSSGKPAIGVGAGNT
PVVIDETADIKRAVASVLMSKTFDNGVICASEQSVVVVDSVYDAVRERFATHG
GYLLQGKELKAVQDVILKNGALNAAIVGQPAYKIAELAGFSVPENTKILIGEVT
VVDESEPFAHEKLSPTLAMYRAKDFEDAVEKAEKLVAMGGIGHTSCLYTDQD
NQPARVSYFGQKMKTARILINTPASQGGIGDLYNFKLAPSLTLGCGSWGGNSIS
ENVGPKHLINKKTVAKRAENMLWHKLPKSIYFRRGSLPIALDEVITDGHKRALI
VTDRFLFNNGYADQITSVLKAAGVETEVFFEVEADPTLSIVRKGAELANSFKPD
VIIALGGGSPMDAAKIMWVMYEHPETHFEELALRFMDIRKRIYKFPKMGVKAK
MIAVTTTSGTGSEVTPFAVVTDDATGQKYPLADYALTPDMAIVDANLVMDMP
KSLCAFGGLDAVTHAMEAYVSVLASEFSDGQALQALKLLKEYLPASYHEGSK
NPVARERVHSAATIAGIAFANAFLGVCHSMAHKLGSQFHIPHGLANALLICNVI
RYNANDNPTKQTAFSQYDRPQARRRYAEIADHLGLSAPGDRTAAKIEKLLAW
LETLKAELGIPKSIREAGVQEADFLANVDKLSEDAFDDQCTGANPRYPLISELK
QILLDTYYGRDYVEGETAAKKEAAPAKAEKKAKKSA

Un peptide issu de la protéine ADHE est de préférence le peptide de séquence SEQ ID N° 2, tel que défini ci-après :

| **Peptide SEQ ID N°** | **Séquence d'acides aminés** | **Position du peptide dans la protéine** |
|---|---|---|
| **SEQ ID N° 2** | DYVEGETAAK | 866-875 |

Un autre exemple d'espèces bactériennes pouvant être quantifiées selon le procédé de l'invention, est constitué par *Staphylococcus aureus* en utilisant la protéine GUAA-STAAN GMP synthase (glutamine-hydrolyzing) comme marqueur de quantification ou un peptide appartenant à cette protéine. La séquence de la protéine GUAA-STAN est la séquence SEQ ID N° 3 :

SEQ ID N° 3 :
MEMAKEQELILVLDFGSQYNQLITRRIREMGVYSELHDHEISIEEIKKMNPKGII
LSGGPNSVYEEGSFTIDPEIYNLGIPVLGICYGMQLTTKLLGGKVERANEREYG
KAIINAKSDELFAGLPAEQTVWMSHSDKVIEIPEGFEVIADSPSTDYAAIEDKKR
RIYGVQFHPEVRHTEYGNDLLNNFVRRVCDCKGQWTMENFIEIEIEKIRQRVG
DRRVLCAMSGGVDSSVVAVLLHKAIGDQLTCIFVDHGLLRKGEGDMVMEQFG
EGFNMNIIRVNAKDRFMNKLKGVSDPEQKRKIIGNEFVYVFDDEASKLKGVDF
LAQGTLYTDVIESGTKTAQTIKSHHNVGGLPEDMEFELIEPINTLFKDEVRKLGI
ELGIPEHLVWRQPFPGPGLGIRVLGEITEDKLEIVRESDAILRQVIREEGLEREIW
QYFTVLPNIQSVGVMGDYRTYDHTVGIRAVTSIDGMTSDFARIDWEVLQKISSR
IVNEVDHVNRVVYDITSKPPSTIEWE

Un peptide issu de la protéine GUAA-STAN est de préférence le peptide de séquence SEQ ID N° 4, tel que défini ci-après :

| **Peptide SEQ ID N°** | **Séquence d'acides aminés** | **Position du peptide dans la protéine** |
|---|---|---|
| **SEQ ID N° 4** | LGIELGIPEHLVWR | 375-388 |

Un autre exemple d'espèces bactériennes pouvant être quantifiées selon le procédé de l'invention, est constitué par *Pseudomonas aeruginosa* en utilisant la protéine A3LLD0_PSEAI Insulin-cleaving metalloproteinase comme marqueur de quantification ou un peptide appartenant à cette protéine. La séquence de la protéine A3LLD0_PSEAI est la séquence SEQ ID N° 5 :

SEQ ID N° 5 :
MTRMPLATASLLALAISLAGCGDDKKAEAPATPAASTQPAAPAAAPAAKVDE
AAAKAVIKNYADLAEATFADALSTAKDLQKAIDAFLAKPDAETLKAAKEAWF
AARTPYSQSEAFRFGNAIIDDWEGQVNAWPLDEGLIDYVAKDYQHALGNPGA
TANIVANTEIQVGEDKIDVKEITGEKLASLNELGGSEANVATGYHAIEFLLWGQ
DLNGTGPGAGNRPATDYAQGKDCTGGHCDRRAAYLKAVTDLLVSDLEYMAG
QWKAGVADNYRAKLEAEPVDTGLRKMFFGMGSLSLGELAGERMKVALEANS
TEDEHDCFSDDTHHTLFFNGKSIRNIYLGEYKRIDGSVVKGPSLADLVAKADA
AANDTLKADLADTEAKLQAIVDSAEKDGVHFDQMIAPDNKDGQQKIRDAIAA
LVKQTGAIEQAAGKLGIQDLKPDNADHEF

Un peptide issu de la protéine A3LLD0_PSEAI est de préférence le peptide de séquence SEQ ID N° 6, tel que défini ci-après :

| **Peptide SEQ ID N°** | **Séquence d'acides aminés** | **Position du peptide dans la protéine** |
|---|---|---|
| **SEQ ID N° 6** | ADAAANDTLK | 363-372 |

A titre d'autres groupes bactériens pouvant être quantifiés selon le procédé de l'invention, on peut citer comme exemple de famille de bactéries, les *Enterobacteriaceae,* en utilisant comme marqueur de quantification la protéine EFG (Elongation Factor G) ou un peptide appartenant à cette protéine. La séquence de la protéine EFG est la séquence SEQ ID N° 7 :

SEQ ID N° 7 :
MARTTPIARYRNIGISAHIDAGKTTTTERILFYTGVNHKIGEVHDGAATMDWM
EQEQERGITITSAATTAFWSGMAKQYEPHRINIIDTPGHVDFTIEVERSMRVLDG
AVMVYCAVGGVQPQSETVWRQANKYKVPRIAFVNKMDRMGANFLKVVNQI
KTRLGANPVPLQLAIGAEEHFTGVVDLVKMKAINWNDADQGVTFEYEDIPAD
MVELANEWHQNLIESAAEASEELMEKYLGGEELTEAEIKGALRQRVLNNEIIL
VTCGSAFKNKGVQAMLDAVIDYLPSPVDVPAINGILDDGKDTPAERHASDDEP
FSALAFKIATDPFVGNLTFFRVYSGVVNSGDTVLNSVKAARERFGRIVQMHAN
KREEIKEVRAGDIAAAIGLKDVTTGDTLCDPDAPIILERMEFPEPVISIAVEPKTK
ADQEKMGLALGRLAKEDPSFRVWTDEESNQTIIAGMGELHLDIIVDRMKREFN
VEANVGKPQVAYRETIRQKVTDVEGKHAKQSGGRGQYGHVVIDMYPLEPGSN
PKGYEFINDIKGGVIPGEYIPAVDKGIQEQLKAGPLAGYPVVDMGIRLHFGSYH
DVDSSELAFKLAASIAFKEGFKKAKPVLLEPIMKVEVETPEENTGDVIGDLSRR
RGMLKGQESEVTGVKIHAEVPLSEMFGYATQLRSLTKGRASYTMEFLKYDEA
PSNVAQAVIEARGK

Un peptide issu de la protéine EFG est de préférence le peptide de séquence SEQ ID N° 8, tel que défini ci-après :

| **Peptide SEQ ID N°** | **Séquence d'acides aminés** | **Position du peptide dans la protéine** |
|---|---|---|
| **SEQ ID N° 8** | AGDIAAAIGLK | 379-389 |

D'autres groupes de microorganismes peuvent être quantifiés avec le procédé selon l'invention. En particulier, certaines protéines connues comme étant relativement ubiquitaires peuvent être utilisées pour quantifier des microorganismes obtenus à partir de cultures pures.

De manière tout à fait intéressante, selon le second objet de l'invention, de telles protéines peuvent également permettre de mesurer le taux d'expression de protéines ou peptides d'intérêt, tels que par exemple des enzymes bactériennes impliquées dans des mécanismes de résistance aux antibiotiques. Les protéines ou peptides jouant le rôle de marqueurs de quantification permettent de corréler le quantité de protéines d'intérêt avec la quantité de bactéries qui les expriment.

C'est le cas par exemple des protéines RL29, RL 22 et RS 19 qui sont des protéines ribosomales, constitutivement exprimées. Ces protéines ou des peptides issus de ces protéines peuvent permettre de quantifier par exemple des bactéries du genre *Enterococcus* et en particulier *Enterococcus faecalis.*

La protéine ribosomale RL29 fait partie de la sous-unité 50S. Elle est impliquée dans l'étape de traduction. La séquence de la protéine RL29 est la séquence SEQ ID N° 9 :

SEQ ID N° 9 :
MKVKEIRELTTAEMLDKEKQLKEELFNLRFQLATGQLENTARIKEVRQSIARIK
TVLREQAN

Un peptide issu de la protéine RL29 est de préférence le peptide de séquence SEQ ID N° 10, tel que défini ci-après :

| **Peptide SEQ ID N°** | **Séquence d'acides aminés** | **Position du peptide dans la protéine** |
|---|---|---|
| **SEQ ID N° 10** | FQLATGQLENTAR | 30-42 |

La protéine ribosomale RL22 fait également partie de la sous-unité 50S. Cette protéine se lie spécifiquement à l'ARN ribosomal 23S. Elle joue un rôle important dans les premières étapes de l'assemblage des deux sous-unités du ribosome. La séquence de la protéine RL22 est la séquence SEQ ID N° 11 :

SEQ ID N° 11 :
MSEQITSAKATAKTVRTSPRKARLVIDLIRGKSVADAISILKFTPNKSAGIIEKVL
MSAVANAENNFDLDVESLVVSEAFVNEGPTMKRFRPRAKGSASPINKRTSHIT
VVVTEK

Un peptide issu de la protéine RL22 est de préférence le peptide de séquence SEQ ID N° 12, tel que défini ci-après :

| **Peptide SEQ ID N°** | **Séquence d'acides aminés** | **Position du peptide dans la protéine** |
|---|---|---|
| **SEQ ID N° 12** | LVIDLIR | 24-30 |

La protéine ribosomale RS19 fait partie de la sous-unité 30S. Cette protéine forme un complexe avec la protéine RS13 se liant fortement à l'ARN ribosomale 16S. La séquence de la protéine RS19 est la séquence SEQ ID N° 13 :

SEQ ID N° 13 :
MGRSLKKGPFVDDHLMKKVEAQQGAEKKKVIKTWSRRSTIFPSFVGFTIAVYD
GRKHVPVYIQEDMVGHKLGEFAPTRTYRGHVADDKKTKR

Un peptide issu de la protéine RS19 est de préférence le peptide de séquence SEQ ID N° 14, tel que défini ci-après :

| **Peptide SEQ ID N°** | **Séquence d'acides aminés** | **Position du peptide dans la protéine** |
|---|---|---|
| **SEQ ID N° 14** | LGEFAPTR | 71-78 |

Chez *Klebsiella pneumoniae,* d'autres protéines peuvent être utilisées comme marqueurs de quantification. C'est le cas, par exemple, la protéine aconitate hydratase (référence Uniprot W9BG99), la protéine alanine-tRNA ligase (référence Uniprot W9BNB5) et la protéine phosphoenolpyruvate phosphotransferase du système PTS (référence Uniprot W1H324).

La séquence de la protéine aconitate hydratase est la séquence SEQ ID N° 15 :

SEQ ID N° 15 :
MSSTLREASKDTLQVNDKTWHYYSLPLAEKQLGEISRLPKSLKVLMENLLRW
QDGDSVTEEDIRALAGWLQQAHADREIAYRPARVLMQDFTGVPAVVDLAAM
REAVKRLGGDTAKVNPLSPVDLVIDHSVTVDRFGDDEAFEDNVRLEMERNHE
RYAFLRWGQQAFSRFSVVPPGTGICHQVNLEYLGRAVWSEEVNGQWMAWPD
TLVGTDSHTTMINGLGVLGWGVGGIEAEAAMLGQPVSMLIPDVVGFKLSGKL
REGITATDLVLTVTQMLRQHGVVGKFVEFYGDGLDTLPLADRATIANMAPEY
GATCGFFPIDDVTLSYMRLSGRSEEQVALVEAYAKAQGMWRQPGDEPVFTST
LALDMSSVEASLAGPKRPQDRVALGDVPKAFAASGELEVNHLQRQRQPVDYT
LNGHHYSLPDGAVAIAAITSCTNTSNPSVLMAAGLLAKKAVERGLQPQPWVK
ASLAPGSKVVSDYLAHAGLTPYLDQLGFNLVGYGCTTCIGNSGPLPEPIEEAIK
KGDLTVGAVLSGNRNFEGRIHPLVKTNWLASPPLVVAYALAGNMNIDLTREPL
GQGKNGEPVYLKDIWPSGEEIARAVEQVSTEMFRKEYAEVFSGTEEWKAIKVE
ASDTYDWQEDSTYIRLSPFFDEMGAEPLPVEDIRGARILAMLGDSVTTDHISPA
GSIKADSPAGRYLQEHGVARRDFNSYGSRRGNHEVMMRGTFANIRIRNEMVP
GVEGGMTRHLPDPEPMAIYDAAMLYKAEGTPLAVIAGKEYGSGSSRDWAAK
GPRLLGIRVVIAESFERIHRSNLIGMGILPLEFPQGVTRKTLRLTGEERIDISNLQS
LQPGATVPVTLTRADGSQEAIPCRCRIDTATELTYYRNDGILHYVIRNML

Un peptide issu de la protéine aconitate hydratase est de préférence le peptide de séquence SEQ ID N° 16, tel que défini ci-après :

| **Peptide SEQ ID N°** | **Séquence d'acides aminés** | **Position du peptide dans la protéine** |
|---|---|---|
| **SEQ ID N° 16** | AFAASGELEVNHLQR | 392-407 |

La séquence de la protéine alanine-tRNA ligase est la séquence SEQ ID N° 17 :

SEQ ID N° 17 :
MSKSTAEIRQAFLDFFHSKGHQVVASSSLVPHNDPTLLFTNAGMNQFKDVFLG
LDKRNYSRATTAQRCVRAGGKHNDLENVGYTARHHTFFEMLGNFSFGDYFK
QDAIKYAWELLTGENWFALPKEKLWVTVYETDDEAFDIWANEVGVPRERIIRI
GDNKGAPFASDNFWQMGDTGPCGPCTEIFFDHGDHIWGGPPGSPEEDGDRYIE
IWNIVFMQFNRQADGTMEPLPKPSVDTGMGLERIAAVLQHVNSNYDIDLFRDL
IASVAKVTGATDLTNKSLRVIADHIRSCAFLVADGVIPSNENRGYVLRRIIRRAI
RHGNMLGAKDTFFWKLVAPLIDVMGSAGDELKQQQAQVEQVLKTEEEQFAR
TLERGLALLDEELSKLKGDTLDGETAFRLYDTYGFPVDLTADVCRERNIKVDE
AGFEAAMEEQRRRARESSGFGADYNAMIRVDGASEFKGYDHLELNGKVTALF
IDGKAVDSVSAGQEAVVILDQTPFYAESGGQVGDKGELKGAGFSFAVSDTQK
YGQAIGHIGKVASGTLKVGDAVQADVDEARRQRIRLNHSATHLMHAALRQVL
GTHVAQKGSLVNDKALRFDFSHFEAMKPEEIRAVEDLVNAQIRRNLAIETNIM
DIDAARASGAMALFGEKYDDRVRVLRMGDFSTELCGGTHAARTGDIGLFRITS
ESGTAAGVRRIEAVTGEGAMAILHAQSDQLNDIAQLLKGDSHNLGEKVRAALE
RTRQLEKELQQLKEQAAAQESANLSSKAEEINGVKLLVSELTGVEPKMLRTMV
DDLKNQLGSTIVVLATVADGKVSLIAGVSKDVTDRVKAGELVGMVAQQVGG
KGGGRPDMAQAGGTDASALPAALASVKGWVSAKL

Un peptide issu de la protéine alanine-tRNA ligase est de préférence le peptide de séquence SEQ ID N° 18, tel que défini ci-après :

| **Peptide SEQ ID N°** | **Séquence d'acides aminés** | **Position du peptide dans la protéine** |
|---|---|---|
| **SEQ ID N° 18** | LLVSELTGVEPK | 773-785 |

La séquence de la protéine phosphoenolpyruvate phosphotransferase du système PTS est la séquence SEQ ID N° 19 :

SEQ ID N° 19 :
MNNQVLINPSNEQIEALRSLQAQVAEEKAELAKLKDLPAITLDGHQVEVCANI
GTVRDVEGAERNGAEGVGLYRTEFLFMDRDALPTEEEQFAAYKAVAEACGSQ
AVIVRTMDIGGDKELPYMNFPKEENPFLGWRAVRIAMDRKEILRDQVRAILRA
SAFGKLRIMFPMIISVEEVRALKKEIEIYKQELRDEGKAFDESIEIGVMVETPAA
ATIARHLAKEVDFFSIGTNDLTQYTLAVDRGNDMISYLYQPMSPSVLNLIKQVI
DASHAEGKWTGMCGELAGDERATLLLLGMGLDEFSMSAISIPRIKKIIRNTNFE
DAKVLAEQALAQPTTDELMTLVNKFIEEKTIC

Un peptide issu de la protéine phosphoenolpyruvate-protein phosphotransferase est de préférence le peptide de séquence SEQ ID N° 20, tel que défini ci-après :

| **Peptide SEQ ID N°** | **Séquence d'acides aminés** | **Position du peptide dans la protéine** |
|---|---|---|
| **SEQ ID N° 20** | SLQAQVAEEK | 19-29 |

Lorsque les marqueurs de quantification des groupes de microorganismes sont d'origine protéique, en amont de la détection par spectrométrie de masse, l'échantillon à analyser est préférentiellement traité au préalable pour générer des peptides à partir de l'ensemble des protéines présentes dans l'échantillon pour fragmenter ces protéines en peptides, par exemple par digestion avec une enzyme protéolytique (protéase), ou par action d'un réactif chimique. En effet, le clivage des protéines peut être fait par un traitement physico-chimique, par un traitement biologique ou par une combinaison des deux traitements. Parmi les traitements utilisables, on peut citer le traitement par des radicaux hydroxyle, notamment avec de l'H2O2. Le traitement par les radicaux hydroxyle provoque une coupure des liaisons peptidiques qui se fait de manière aléatoire sur n'importe quelle liaison peptidique de la protéine. La concentration en radicaux hydroxyle conditionne le nombre de clivages opérés et donc la longueur des fragments peptidiques obtenus. D'autres traitements chimiques peuvent également être utilisés comme, par exemple, le traitement au bromure de cyanogène (CNBr) qui scinde spécifiquement les liaisons peptidiques au niveau du groupe carboxylique des résidus méthionyles. Il est également possible de réaliser un clivage acide partiel au niveau des résidus aspartyle par chauffage à 1000°C d'une solution de protéines dans de l'acide trifluoroacétique.

Le traitement des protéines par digestion enzymatique est néanmoins préféré par rapport au traitement physico-chimique car il préserve davantage la structure des protéines, et est plus facile à contrôler. Par « digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Chaque protéase reconnaît généralement une séquence d'acides aminés au sein desquels elle effectue toujours la même coupure. Certaines protéases reconnaissent un seul acide aminé ou une séquence de deux acides aminés entre lesquels elles opèrent un clivage, d'autres protéases ne reconnaissent que des séquences plus longues. Ces protéases peuvent être des endoprotéases ou des exoprotéases. Parmi les protéases connues on peut citer, comme décrit dans WO-A-2005/098017 :
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH2 des résidus aromatiques (Phe, Tyr et Trp), la protéase V8 de la souche V8 de Staphylococcus aureus qui clive la liaison peptidique au niveau du groupe carboxylique du résidu Glu ;
- les enzymes non-spécifiques comme la thermolysine provenant de la bactérie *Bacillus thermoproteolyticus* qui hydrolyse la liaison peptidique du groupe NH2 des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles. Elles peuvent également être utilisées de façon successive. Dans le cadre de l'invention, la digestion de l'échantillon est, de préférence, réalisée par action d'une enzyme protéase, par exemple la trypsine.

La génération de peptides à l'aide d'un réactif chimique ou d'une protéase, peut être obtenue par simple réaction en solution. Elle peut également être mise en œuvre avec un four à micro-ondes [11], ou sous pression [12], ou bien encore avec un dispositif à ultrasons [13]. Dans ces trois derniers cas, le protocole sera beaucoup plus rapide. Parmi les peptides ainsi obtenus, les peptides spécifiques de la protéine, sont nommés peptides protéotypiques. Ce sont eux qui seront dosés par spectrométrie de masse. Selon un mode de réalisation de l'invention, les marqueurs de quantification sont des peptides correspondants à une protéine du groupe bactérien à quantifier. En particulier, lesdites protéines sont digérées en peptides, de préférence par une enzyme, de préférence encore par la trypsine.

De même, l'échantillon contenant des marqueurs de quantification d'origine protéique peut également être préalablement traité à des fins de purification. Lorsque les marqueurs sont d'origine protéique, ce traitement préalable de purification peut être mis en œuvre avant ou après l'étape de génération de peptides tels que décrits précédemment.

Le traitement préalable de purification d'échantillon est largement connu de l'homme du métier et pourra notamment mettre en œuvre des techniques de centrifugation, de filtration, d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al. [14], ou H. Keshishian et al. [15]. Dans ces publications, le milieu chromatographique peut être en colonne ou en cartouche (extraction en phase solide).

La fraction électrophorétique ou chromatographique (ou le temps de rétention en chromatographie mono ou multidimensionnelle) des peptides protéotypiques est caractéristique de chaque peptide et la mise en œuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatographie, pour le fractionnement des peptides, consiste à purifier spécifiquement les N-glycopeptides ([16] et demande de brevet WO-A-2008/066629). Néanmoins, une telle purification ne permet que la quantification des peptides ayant subi une modification post-traductionnelle de type N-glycosylation. Or toutes les protéines ne sont pas glycosylées, ce qui limite donc son utilisation.

La spectrométrie de masse à mettre en œuvre dans le procédé de l'invention est largement connue de l'homme du métier comme un outil puissant pour l'analyse, la détection et la quantification de différents types de molécules. De façon générale, tout type de molécule pouvant être ionisée peut être détectée et quantifiée en fonction de sa masse moléculaire à l'aide d'un spectromètre de masse. Selon la nature de la molécule à détecter, d'origine protéique ou métabolique, certaines technologies de spectrométrie de masse peuvent être plus adaptées. Néanmoins, quelle que soit la méthode de spectrométrie de masse utilisée pour la détection, cette dernière comprend une étape d'ionisation de la molécule cible en ions dits moléculaires, dans le cas présent une étape d'ionisation des marqueurs de quantification, et une étape de séparation des ions moléculaires obtenus en fonction de leur masse.

Tous les spectromètres de masse comportent donc :
- une source d'ionisation destinée à ioniser les marqueurs présents dans l'échantillon à analyser, c'est-à-dire à conférer une charge positive ou négative à ces marqueurs;
- un analyseur de masse destiné à séparer les marqueurs ionisés, ou ions moléculaires, en fonction de leur ratio masse sur charge (m /z) ;
- un détecteur destiné à mesurer le signal produit soit directement par les ions moléculaires, soit par des ions produits à partir des ions moléculaires. Ce signal peut être utilisé de façon qualitative ou quantitative, comme détaillés ci-après.

L'étape d'ionisation nécessaire pour la mise en œuvre d'une spectrométrie de masse peut être mise en œuvre par tout procédé connu de l'homme du métier. La source d'ionisation permet d'amener les molécules à doser sous un état gazeux et ionisé. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :
- l'ionisation électronique (EI), l'ionisation chimique (CI) et la désorption-ionisation chimique (DCI)
- le bombardement par atomes rapides (FAB), atomes métastables (MAB) ou ions (SIMS, LSIMS)
- le couplage plasma inductif (ICP)
- l'ionisation chimique à pression atmosphérique (APCI) et la photoionisation à pression atmosphérique (APPI)
- l'électronébulisation ou électrospray (ESI)
- l'ionisation par désorption électrospray (DESI) ou nano-électrospray (nanoDESI)
- l'ionisation électrospray par ablation laser (LAESI)
- l'ionisation par évaporation rapide (REIMS)
- l'ionisation-désorption par interaction avec espèces métastables (DART).

Notamment, l'ionisation peut être mise en œuvre comme suit : l'échantillon contenant les molécules cibles est introduit dans une source d'ionisation, où les molécules sont ionisées à l'état gazeux et ainsi transformées en ions moléculaires qui correspondent aux molécules initiales. Une source d'ionisation de type électrospray (ESI pour ElectroSpray Ionisation) permet d'ioniser une molécule tout en la faisant passer d'un état liquide à un état gazeux. Les ions moléculaires obtenus correspondent alors aux molécules présentes à l'état liquide, avec en mode positif un, deux, voire trois protons supplémentaires ou plus et sont donc porteurs de une, deux, voire trois charges ou plus. Par exemple, lorsque la molécule cible est une protéine, une ionisation des peptides protéotypiques obtenus après fractionnement de la protéine cible, grâce à une source de type Electrospray fonctionnant en mode positif, conduit à des ions polypeptidiques à l'état gazeux, avec un, deux, voire trois protons supplémentaires ou plus et qui sont donc porteurs de une, deux, voire trois charges ou plus, et permet un passage d'un état liquide à un état gazeux [17]. Ce type de source est particulièrement bien adapté, lorsque les molécules cibles ou peptides protéotypiques obtenus sont préalablement séparées par chromatographie liquide en phase inverse. Néanmoins, le rendement d'ionisation des molécules présentes dans l'échantillon peut varier en fonction de la concentration et de la nature des différentes espèces en présence. Ce phénomène se traduit par un effet matrice bien connu de l'homme de l'art.

L'analyseur de masse dans lequel est mis en œuvre l'étape de séparation des marqueurs ionisés en fonction de leur rapport masse/charge (m/z) est tout analyseur de masse connu de l'homme du métier. On peut citer les analyseurs basse résolution, du type quadripôle ou quadrupôle (Q), piège à ions 3D (IT) ou linéaire (LIT), également appelés trappe ionique, et les analyseurs haute résolution, permettant de mesurer la masse exacte des analytes et qui utilisent notamment le secteur magnétique couplé à un secteur électrique, le temps de vol (TOF), l'orbitrap, la résonance cyclotronique à transformée de Fournier (FT-ICR) .

Lors de la séparation des ions moléculaires en fonction de leur ratio m/z, plusieurs étapes de séparation MS successives peuvent être menées. Lorsque deux étapes de séparation MS successives sont réalisées, l'analyse est appelée MS/MS ou MS². Lorsque trois étapes de séparation MS successives sont réalisées, l'analyse est appelée MS/MS/MS ou MS³ et plus généralement, lorsque n étapes de séparation MS successives sont réalisées, l'analyse est appelée MSⁿ.

Parmi les techniques mettant en œuvre plusieurs séparations successives, les modes SRM (Selected Reaction Monitoring) en cas de détection ou dosage d'une seule molécule cible, ou bien MRM (Multiple Reaction Monitoring) en cas de détection ou dosage de plusieurs molécules cibles, sont des utilisations particulières de séparation MS². De même le mode MRM³ est une utilisation particulière de séparation en MS/MS/MS. On parle alors de spectrométrie de masse ciblée.

Dans le cas d'une détection en mode MS simple, c'est le rapport masse/charge des ions moléculaires obtenus qui est corrélé à la molécule cible à détecter.

Dans le cas d'une détection en mode MS/MS, essentiellement deux étapes sont ajoutées, par rapport à un dosage MS qui sont :
une fragmentation des ions moléculaires, alors appelés ions précurseurs, pour donner des ions dit ions fragments de 1ère génération, et
une séparation des ions dit ions fragments de 1ère génération en fonction de leur masse (m/z)₂, le rapport (m/z)₁ correspondant au rapport (m/z) des ions précurseurs.

C'est alors le rapport masse/charge des ions fragments de 1ère génération ainsi obtenus qui est corrélé à la molécule cible à détecter. Par ion fragment de première génération, on entend un ion issu de l'ion précurseur, suite à une étape de fragmentation et dont le rapport masse sur charge m/z est différent de l'ion précurseur.

Les couples (m/z)₁ et (m/z)₂ sont baptisés transitions et sont représentatifs des ions caractéristiques à détecter.

Le choix des ions caractéristiques qui sont détectés pour être corrélés à la molécule cible est effectué par l'homme du métier selon les méthodes standards. Leur sélection conduira avantageusement aux dosages les plus sensibles, les plus spécifiques et les plus robustes possibles, en termes de reproductibilité et fiabilité. Dans les méthodes développées pour la sélection de peptides protéotypiques (m/z)₁, et de fragment de première génération (m/z)₂, le choix est essentiellement basé sur l'intensité de la réponse. Pour plus de détails, on pourra se référer à V. Fusaro et al. [18]. Des logiciels commerciaux, tels que les logiciels MIDAS et MRM Pilote d'Applied Biosytems ou encore MRMaid [19] pourront être utilisés par l'homme de l'art pour lui permettre de prédire tous les couples de transitions possibles. Il pourra également être fait appel à une base de données nommée PeptideAtlas, construite par F. Desiere et al. [20] pour compiler l'ensemble des transitions MRM de peptides décrites par la communauté scientifique. Cette base PeptideAtlas est disponible en accès libre sur internet. Pour des molécules non protéiques, il est également possible d'utiliser des bases de données, telles que par exemple celle accessible au travers du logiciel Cliquid de la société AB Sciex (Framingham, Massachusetts, Etats Unis d'Amérique).

Une approche alternative pour sélectionner les peptides protéotypiques, (m/z)₁ et (m/z)₂, consiste à utiliser les spectres de fragmentation MS/MS obtenus à l'occasion d'autres travaux. Ces travaux peuvent être, par exemple, les phases de découverte et d'identification des biomarqueurs par analyse protéomique. Cette approche a été proposée par Thermo Scientific lors de réunion utilisateurs [19]. Elle permet de générer une liste de transitions candidates à partir des peptides identifiés expérimentalement par le logiciel SIEVE (Thermo Scientific). Certains critères ont été détaillés par J. Mead et al. [19] pour le choix des ions (m/z)₁ et (m/z)₂ et sont détaillés ci-après :
Les peptides avec des sites de clivage interne, c'est-à-dire avec de la Lysine ou de l'Arginine interne, doivent être évités, sauf si la Lysine ou l'Arginine est suivie par de la Proline,
Les peptides avec de l'Asparagine ou de la Glutamine doivent être évités car ils peuvent se désaminer,
Les peptides avec de la Glutamine ou de l'Acide Glutamique en N-terminal doivent être évités car ils peuvent se cycliser spontanément,
Les peptides avec de la Méthionine doivent être évités car ils peuvent être oxydés,
Les peptides avec de la Cystéine doivent être évités car ils peuvent être modifiés de façon non reproductible lors d'une éventuelle étape de dénaturation, réduction et blocage des fonctions thiols.

Les peptides avec de la Proline peuvent être considérés comme favorables parce qu'ils produisent généralement des fragments intenses en MS/MS avec un seul pic très majoritaire. Cependant, un seul fragment très majoritaire ne permet pas de valider l'identité de la transition dans un mélange complexe. En effet, seule la présence simultanée de plusieurs fragments caractéristiques permet de vérifier que l'ion précurseur recherché est bien détecté.

Les peptides ayant une Proline adjacente au C-terminal (position n-1) ou en seconde position par rapport au C-terminal (position n-2) sont à éviter car, dans ce cas, la taille du peptide fragment de première génération est généralement considérée comme trop petite pour être suffisamment spécifique.

La sélection de fragments ayant une masse supérieure au précurseur est à privilégier pour favoriser la spécificité. Pour cela, il faut sélectionner un ion précurseur dichargé et sélectionner l'ion fragment de première génération le plus intense ayant une masse supérieure au précurseur, c'est à dire un ion fragment de première génération monochargé.

La fragmentation des ions précurseurs sélectionnés est mise en œuvre dans une cellule de fragmentation telle que les modèles de type triple quadripôle [21], ou de type trappe ionique [22], ou encore de type temps de vol (TOF) [23], lesquels permettent également la séparation des ions. La ou les fragmentations seront classiquement réalisées par collision avec un gaz inerte tel que l'argon ou l'azote, au sein d'un champ électrique, par photo-excitation ou photodissociation à l'aide d'une source lumineuse intense, collision avec des électrons ou espèces radicalaires, par application d'une différence de potentiel, par exemple dans un tube de temps de vol, ou par tout autre mode d'activation. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser. A titre d'exemple, il est possible de faire varier l'énergie de collision entre 5 et 180 e-V en q2 dans un spectromètre de masse QTRAP^{®} 5500 de la société AB Sciex (Framingham, Massachusetts, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse QTRAP^{®} 5500.

Enfin, la détection des ions caractéristiques sélectionnés se fait de façon classique, notamment grâce à un détecteur et à un système de traitement. Le détecteur collecte les ions et produit un signal électrique dont l'intensité dépend de la quantité d'ions collectée. Le signal obtenu est ensuite amplifié pour qu'il puisse être traité informatiquement. Un ensemble informatique de traitement des données permet de transformer les informations reçues par le détecteur en spectre de masse.

Le principe du mode SRM, ou encore du mode MRM, est de sélectionner spécifiquement un ion précurseur, de le fragmenter, puis de sélectionner spécifiquement l'un de ses ions fragments. Pour de telles applications, des dispositifs du type triple quadripôle ou des hybrides triple quadripôle à trappe ionique sont généralement utilisés.

Dans le cas d'un dispositif triple quadripôle (Q1q2Q3) utilisé en mode MS², en vue du dosage ou de la détection d'une protéine cible, le premier quadripôle (Q1) permet de filtrer les ions moléculaires, correspondant aux peptides protéotypiques caractéristiques de la protéine à doser et obtenus lors d'une étape antérieure de digestion, en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure. L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon dans q2. Les ions fragments de première génération sont transmis dans un troisième quadripôle (Q3) qui filtre les ions fragments de première génération en fonction d'un ratio masse sur charge spécifique, ratio appelé (m/z)₂. Seuls les ions fragments de première génération ayant le ratio masse/charge d'un fragment caractéristique du peptide protéotypique recherché (m/z)₂ sont transmis dans le détecteur pour être détectés, voire quantifiés.

Ce mode de fonctionnement présente une double sélectivité, en relation avec la sélection de l'ion précurseur d'une part et de la sélection de l'ion fragment de première génération d'autre part. La spectrométrie de masse en mode SRM ou MRM est donc avantageuse pour la quantification

Lorsque la spectrométrie de masse mise en œuvre dans le procédé de l'invention est une spectrométrie de masse en tandem (MS², MS³, MS⁴ ou MS⁵), plusieurs analyseurs de masse peuvent être couplés entre eux. Par exemple, un premier analyseur sépare les ions, une cellule de collision permet de fragmenter les ions, et un second analyseur sépare les ions fragments. Certains analyseurs, comme les pièges à ions ou le FT-ICR, constituent plusieurs analyseurs en un et permettent de fragmenter les ions et d'analyser les fragments directement.

Selon des modes de réalisation préférés de l'invention, le procédé de l'invention comprend une ou plusieurs des caractéristiques suivantes :
- la spectrométrie de masse, mise en œuvre pour la quantification d'un groupe bactérien, est une spectrométrie de type MS/MS, ce qui a pour avantage de générer un fragment spécifique de la molécule à détecter ou à quantifier, et ainsi d'apporter une grande spécificité à la méthode de dosage ;
- la spectrométrie MS/MS est une méthode de spectrométrie de masse quantitative, pouvant être une méthode de DIA (Data Independent Analysis) [25] comprenant entre autre, le SWATH (Sequential Windows Acquisition of All THeoretical fragment) [26], ou la MS^{E} [27], une méthode DDA (Data Dependent Acquisition), une méthode de PRM (Parallel Reaction Monitoring), de SRM ou de MRM.

La MRM, qui a pour avantage d'utiliser un temps de cycle d'analyse dans le spectromètre de masse de quelques dizaines de millisecondes, qui permet de détecter ou de quantifier avec une grande sensibilité, et de façon multiplexée, un grand nombre de molécules différentes ;

Le DIA qui a pour avantage de permettre l'identification de molécules et la quantification avec une bonne sensibilité, et spécificité, et avec une capacité de multiplexage encore plus importante.

Un des avantages de l'utilisation de la spectrométrie de masse réside en ce qu'elle est particulièrement utile pour quantifier des molécules, dans le cas présent les marqueurs de quantification d'au moins un groupe bactérien. Pour ce faire, on utilise l'intensité de courant détectée, laquelle est proportionnelle à la quantité de molécule cible. L'intensité de courant ainsi mesurée pourra servir de mesure quantitative permettant de déterminer la quantité de molécule cible présente, laquelle est caractérisée par son expression en unités du Système International (SI) de type mol/m3 ou kg/m3, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative.

Un calibrage est néanmoins nécessaire pour pouvoir corréler l'aire du pic mesurée, correspondant à l'intensité de courant induit par les ions détectés, à la quantité de molécule cible à doser. Pour cela, les calibrages classiquement utilisés en spectrométrie de masse pourront être mis en œuvre, dans le cadre de l'invention. Les dosages MRM sont classiquement calibrés à l'aide de standards externes ou, de préférence, à l'aide de standards internes tels que décrits par T. Fortin et al. [14]. Dans le cas où la molécule cible est un peptide protéotypique, permettant de doser une protéine d'intérêt, la corrélation entre la mesure quantitative et la quantité de peptide protéotypique cible, et par la suite de la protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe), ou de façon préférentielle, par étalonnage interne en utilisant un peptide lourd, comme standard interne, par exemple conformément aux méthodes AQUA, QconCAT ou PSAQ détaillées ci-après. Par « peptide lourd », on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N)*.*

L'utilisation de peptides lourds, comme standards internes (AQUA), a également été proposée dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N)*.* Le peptide artificiel, encore appelé peptide lourd ou peptide AQUA, ainsi synthétisé a rigoureusement les mêmes propriétés physicochimiques que le peptide naturel, encore appelé peptide léger ; la seule différence entre ces deux peptides réside dans leur masse, plus élevée pour le peptide lourd et plus faible pour le peptide léger. Il est généralement ajouté, à une concentration donnée, à l'échantillon, en amont du dosage par spectroscopie de masse, par exemple entre le traitement entrainant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. [35] sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entrainant le clivage des protéines et avant les étapes de fractionnement des protéines, de dénaturation, de réduction puis de blocage des fonctions thiols des protéines, si celles-ci sont présentes. Le standard QconCAT subit donc le même cycle de traitement entrainant le clivage des protéines que la protéine naturelle, ce qui permet de tenir compte du rendement de l'étape de traitement entrainant le clivage des protéines. En effet, le traitement, notamment par digestion, de la protéine naturelle peut ne pas être complet. Dans ce cas l'utilisation d'un standard AQUA conduirait à sous-estimer la quantité de protéine naturelle. Pour un dosage absolu, il peut donc être important de tenir compte des rendements de traitement entrainant le clivage des protéines. Cependant, V. Brun et al. [36] ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. [36] ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée ex-vivo avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.

L'ensemble de ces techniques, à savoir AQUA, QconCAT ou PSAQ ou toute autre technique de calibrage, utilisée dans des dosages par spectrométrie de masse et en particulier dans les dosages MRM ou MS, pourront être mises en œuvre pour effectuer le calibrage, dans le cadre de l'invention.

Dans le cadre de l'invention, une droite de calibration externe pourra être réalisée, avec une gamme de concentrations variables connues du groupe de microorganismes à quantifier. La quantification d'au moins un groupe de microorganismes se fera par interpolation de la quantité du marqueur d'au moins un groupe de microorganismes à quantifier dans un échantillon, sur une ou plusieurs droites de calibrations externes du ou des marqueurs protéiques ou peptidiques desdits groupes de microorganismes à quantifier. La gamme de calibration externe peut par exemple être réalisée avec des échantillons de culture pures du au moins un groupe de microorganismes à quantifier.

La quantité de bactéries contenue dans une gamme de calibration est caractérisée par son expression en nombre de bactéries par litre ou en g/l ou par un multiple ou un sous-multiple de ces unités, ou par les dérivées usuelles de ces unités, y compris leurs multiples ou leurs sous-multiples. Ainsi, l'homme du métier exprime fréquemment la quantité de bactéries dans un échantillon liquide en CFU/mL ou par un multiple ou un sous-multiple de cette unité. CFU est l'acronyme anglais de « Colony-Forming Units » pour Unité Faisant Colonie. Cette unité usuelle correspond au nombre de bactéries ayant formées une colonie après culture sur une boite de Petri (à condition que l'isolement des colonies soit suffisant pour que chaque colonie soit clairement séparée des colonies adjacentes).

L'homme du métier utilise également l'unité de McFarland (McF). Cette unité correspond à la mesure de la turbidité d'une suspension bactérienne mesurée à 600 nm par néphélométrie, calibré par des standards McFarland réalisés avec un mélange de chlorure de baryum et d'acide sulfurique réalisé de la façon suivante (TABLEAU 1):

**TABLEAU 1**

| | | | | | |
|---|---|---|---|---|---|
| Numero du standard McFarland | 0.5 | 1 | 2 | 3 | 4 |
| Volume de chlorure de Barium à 1.0% ene au (ml) | 0.05 | 0.1 | 0.2 | 0.3 | 0.4 |
| Volume d'acide sulfurique à 1.0% ene au (ml) | 9.95 | 9.9 | 9.8 | 9.7 | 9.6 |
| Densité cellulaire approximative (108 CFU/mL) | 1.5 | 3.0 | 6.0 | 9.0 | 12.0 |
| Pourcentage de transmittance à 600nm | 74.3 | 55.6 | 35.6 | 26.4 | 21.5 |
| Absorbance à 600nm | 0.08 à 0.1 | 0.257 | 0.451 | 0.582 | 0.669 |

L'unité de McFarland est ainsi proportionnelle à une quantité de bactéries, mais il convient de noter que cette correspondance dépend de l'espèce bactérienne, du fait d'une turbidité propre à chaque espèce. Pour utiliser de façon quantitative un dosage bactérien effectué en McF, il convient donc d'étalonner au préalable la quantité observée en McF avec des solutions dont la quantité de bactéries est caractérisées, par exemple, par une dose en CFU/ml.

Dans le cadre de l'invention, la quantité de bactéries peut également être mesurée de façon relative et utilisée pour effectuer une quantification relative. Dans ce cas, il n'est pas nécessaire de corréler une mesure caractérisant la quantité de bactéries à une quantité de bactéries exprimée dans l'une des unités caractéristiques mentionnées ci-dessous. De façon avantageuse, le procédé selon l'invention permet de mesurer au sein de la même analyse des signaux correspondant à des peptides, ou des protéines caractéristiques respectivement de la quantité de microorganismes et de la quantité du ou des peptides d'intérêt et/ou du ou des protéines d'intérêt. Le ratio entre le signal des peptides ou des protéines d'intérêt et des peptides, ou des protéines caractéristiques de la quantité de microorganismes, permet d'obtenir une quantification relative du taux d'expression des peptides et/ou des protéines d'intérêt sans avoir à utiliser une droite d'étalonnage comme mentionné ci-dessus.

Les exemples qui suivent sont fournis uniquement à titre illustratif et ne constituent en rien une limitation de l'invention.

### EXEMPLES

### Exemple 1 : Quantification bactéries issues de culture pures, par identification des peptides représentatifs par MRM

### 1. Mise en culture de bactéries sur milieux de culture gélosés :

Les milieux de culture optimaux et les conditions de culture optimales sont différents selon les espèces de microorganisme.

Pour les microorganismes utilisés, la milieu de culture utilisé est une gélose Columbia au sang de mouton (gélose COS - référence bioMérieux 43041). Un isolement est réalisé sur ce milieu de culture puis ce dernier est incubé pendant 18 à 24 h à 37°C, en atmosphère aérobie ou anaérobie selon les espèces.

### 2. Mise en culture des bactéries en bouillon :

a) Une ou plusieurs colonies isolées selon le protocole décrit dans l'étape 1, sont sélectionnées.
b) Ces colonies sont mises en suspension dans un bouillon TSB (référence bioMérieux 42100) de 9mL, puis mises en culture à 37°C pendant 15-18 heures.
c) Une fraction de la suspension obtenue à l'étape b) est prélevée à l'aide d'une pipette pasteur stérile et mise dans un tube contenant 3,0 mL de solution saline stérile aqueuse (à 0,45-0,50 % de NaCl, de pH 4,5 à 7,0). La concentration en bactéries dans cette nouvelle suspension est ensuite ajustée entre 0,50 et 0,8 McFarland avec un DENSIMAT (commercialisé par la demanderesse) étalonné (cette densité permet d'obtenir une quantité entre 1,5×10⁸ et 2,4×10⁸ CFU/mL chez *E. coli*)*.* Cette solution est notée « H0 ».
d) A partir d'1 mL de solution H0, six dilutions sérielles au 1/10^{ème} sont alors effectuées dans des tubes contenant 9mL Trypton Sel étalonnés (commercialisé par la demanderesse).
e) Un étalement de 100µL de chacun des deux derniers tubes de dilution est ensuite réalisé sur trois géloses COS pour déterminer la concentration bactérienne de la solution « H0 ». Les géloses sont mises en culture pendant 12-18 heures à 37°C.
f) 1mL de la dilution au 1/100^{ème} de la solution « H0 » préparée à l'étape d) est à nouveau diluée dans 10 tubes contenant 9mL de bouillons TSB.
g) Neuf des dix tubes de bouillon TSB sont mis dans un incubateur-agitateur à 37°C. 1mL du bouillon contenu dans le dernier tube, noté « H1 », est prélevé et mis dans un tube de 1,5mL afin d'être utilisé pour l'obtention de protéines digérées à partir des bactéries en suspension.
h) Régulièrement dans la journée (environ une fois par heure), chacun des neuf tubes de bouillon TSB est sorti de l'incubateur. Il est noté « Hx » (avec × allant de 2 à 10). Chacun des tubes subit le même protocole que le tube « H1 », comme décrit à l'étape g).

### 3. Obtention des protéines digérées :

Classiquement le protocole suivant est mis en œuvre en 15 étapes :
a) Prélèvement d'1 mL de Bouillon « Hx » cité précédemment.
b) Centrifugation 10minutes à 6000G.
c) Elimination du surnageant.
d) Reprise du culot dans 150µL de tampon bicarbonate 50mM pH=8.
e) Ajout de 10 µL d'un pool de peptides lourds (peptides correspondant aux peptides protéotypiques, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N))*.*
f) Ajout de dithiothréitol (DTT) pour obtenir une concentration finale de 5mM.
g) Ajout de 150mg de billes de verre de 1mm de diamètre et 50mg de billes de zirconium/silice de 0,1mm de diamètre (Biospec Product (Bartlesville, OK, USA)).
h) Lyse des bactéries et réduction des protéines en mettant les tubes dans une sonde à ultrasons pendant 5 minutes (95°C).
i) Refroidissement des tubes dans la glace pendant 1 minute.
j) Ajout d'iodoacétamide pour obtenir une concentration finale de 12,5 mM.
k) Alkylation pendant 5minutes à température ambiante et à l'obscurité.
l) Ajout de 200 µg de trypsine.
m) Digestion à 50°C pendant 15 minutes dans un Thermomixer^{®} à 850 rpm.
n) Ajout d'acide formique à 0,5% jusqu'à obtention d'un pH inférieur à 4 pour stopper la réaction.
o) Centrifugation 20 minutes à 10000G.

### 4. Caractérisation d'échantillons de bactéries

Chaque échantillon est traité selon le protocole du paragraphe 3, puis un volume de 50µl de protéines digérées est injecté et analysé selon les conditions suivantes :
- Chaîne chromatographique Nexera de la société SHIMADZU (Kyoto, Japon).
- Colonne Waters (Waters, Saint-Quentin en Yvelines, France) BEH C18, 2,1mm de diamètre interne, 100mm de long, taille de particule de 3,5µm.
- Solvant A : H2O + 0,1% acide formique.
- Solvant B : ACN + 0,1% acide formique.
- Gradient HPLC défini au **TABLEAU 2** ci-après :

**TABLEAU 2**

| **Temps** | **Débit (µl)** | **Solvent A (%)** | **Solvent B (%)** |
|---|---|---|---|
| 0 | 300 | 98 | 2 |
| 3 | 300 | 98 | 2 |
| 25 | 300 | 67 | 33 |
| 25.1 | 300 | 0 | 100 |
| 35 | 300 | 0 | 100 |
| 35.1 | 300 | 98 | 2 |
| 45 | 300 | 98 | 2 |

L'éluât en sortie de colonne chromatographique est directement injecté dans la source d'ionisation du spectromètre de masse de type QTRAP^{®} 5500 de la société AB Sciex (Framingham, Massachusetts,, Etats Unis d'Amérique).
- Les peptides, issus de la digestion des protéines de la bactérie, sont analysés par le spectromètre de masse en mode MRM. Les peptides suivis et détectés permettent l'identification d'un groupe de microorganisme grâce à leur caractère spécifique d'un groupe de microorganismes. Pour cela, le ou les fragments indiqués dans le **TABLEAU 3** sont détectés.

**TABLEAU 3**

| **Espèce** | **Référence Uniprot de la protéine** | **Séquence du peptide** | **Ion fragment** | **m/z₁ Q1** | **m/z₂ Q3** |
|---|---|---|---|---|---|
| *Psedomonas aeruginosa* | A3LLD0 | ADAAANDTLK | y6 monochargé | 495.249 | 661.352 |
| | | | y7 monochargé | 495.249 | 732.389 |
| | | | y8 monochargé | 495.249 | 803.426 |
| *Escherichia coli* | ADHE | DYVEGETAAK | y6 monochargé | 541.754 | 576.299 |
| | | | y7 monochargé | 541.754 | 705.341 |
| | | | y8 monochargé | 541.754 | 804.41 |
| *Staphylococcus aureus* | GUAA | LGIELGIPEHLVWR | y3 monochargé | 544.64 | 460.266 |
| | | | y7 dichargé | 544.64 | 468.756 |
| | | | y7 monochargé | 544.64 | 936.505 |
| *Enterobacteriaceae* | EFG | AGDIAAAIGLK | y6 monochargé | 500.295 | 572.377 |
| | | | y7 monochargé | 500.295 | 643.414 |
| | | | Y5monochargé | 500.295 | 501.339 |

Le ratio de l'aire sous le pic représentatif du peptide étudié par rapport à l'aire sous le pic du peptide lourd de synthèse correspondant est considéré comme la réponse observée, et sera mis en corrélation avec la quantité de bactéries mesurée.

Les autres paramètres machine utilisés sont les suivants :

| | |
|---|---|
| • Type de balayage: | MRM |
| • Polarité: | Positive |
| • Source d'ionisation: | Turbo V^{™} (AB Sciex) |
| • Réglage Q1: | Filtrage avec résolution unitaire |
| • Réglage Q3: | Filtrage avec résolution unitaire |
| • Pause inter-scan: | 3.00 msec |
| • Vitesse de balayage: | 10 Da/s |
| • Gaz rideau: | 50,00 psi |
| • Tension de cône: | 5500,00 V |
| • Température de source: | 550,00 °C |
| • Gaz de nébulisation: | 50,00 psi |
| • Gaz chauffant: | 50,00 psi |
| • Remplissage dynamique: | activé |
| • Potentiel d'entrée avant Q0 (EP): | 10,00 V |
| • Potentiel en sortie de cellule de collision : | 15 V (en anglais cell exit potential (CXP)) |
| • Temps de cycle total: | 1.2 sec |

### 5. Identification des peptides représentatifs de la quantité de bactéries :

### a. Peptides dont l'expression ne dépend pas de la phase de culture

Différentes souches des espèces listées ci-dessous, provenant du souchier de la demanderesse) sont traitées et analysées comme décrit précédemment (paragraphes 2, 3 et 4).
*Pseudomonas aeruginosa* : Souche 1 et souches 4 à 6
*Escherichia coli* : souches 2 et 14
*Staphylococcus aureus* : souches 3 et 23

Les coefficients de corrélation entre la mesure de la quantité de bactéries et la quantité de peptide mesurée en masse, au cours du temps, c'est-à-dire quelle que soit la phase de croissance de la bactérie sont représentés dans le **TABLEAU 4** ci-dessous.

**TABLEAU 4**

| **Espèce** | **Protéine** | **Peptide** | **R²** |
|---|---|---|---|
| *P.aeruginosa* | A3LLD0 | ADAAANDTLK | 0.93 |
| *E.coli* | ADHE | DYVEGETAAK | 0.98 |
| *Enterobacteriaceae* | EFG | AGDIAAAIGLK | 0.98 |
| *S.aureus* | GUAA | LGIELGIPEHLVWR | 0.97 |

Les résultats sont présentés sur les figures 1 à 4. Ces figures illustrent la corrélation entre la quantité de bactéries mesurée par culture quantitative (dénombrement sur boite de Petri après dilutions successives) et la quantité de bactéries mesurée par spectrométrie de masse en mesurant l'aire sous le pic chromatographique d'un peptide spécifique de l'espèce bactérienne à quantifier. Cette corrélation est illustrée dans cet exemple, en fonction du temps de croissance de la bactérie.

Pour les peptides représentés ici, la quantité mesurée dépend uniquement de la quantité de bactéries en présence, et non de la phase de croissance dans laquelle se trouve la bactérie.

### b. Peptides dont l'expression ne dépend pas de la souche utilisée

Après mise en culture de souche conformément à ce qui décrit au paragraphe 1 *supra,* une suspension de quelques colonies est réalisée dans 9 mL de bouillon TSB , et mis à incuber entre 12h et 15h à 37°C.

La concentration en bactéries de la solution est réalisée par mesure de McFarland avec un Densimat étalonné.

Les solutions sont comprises entre 3 et 7 McFarland cette densité permet d'obtenir une quantité entre 9×10⁸ et 2,1×10⁹ CFU/mL chez *E. coli*)

Sept dilutions successives au 1/10^{ème} sont réalisées et 100µL de chacune des deux dernières dilutions sont étalées sur trois géloses COS. Les géloses sont mise à l'étuve à 37°C pendant 18h dans le but de permettre une quantification exacte du nombre de CFU/ml présent dans le bouillon de départ.

Le protocole décrit *supra,* utilisé pour obtenir les protéines digérées est reproduit avec 1mL du bouillon de départ ainsi que les cinq premiers tubes de dilution.

Six points de gamme de chacune des souches utilisées ont été suivis par la méthode de caractérisation propre à chaque groupe bactérien. Autrement dit, chaque espèce est suivi en MRM par détection des peptides propres à l'espèce ou à la famille.

Liste des échantillons analysés :
*Pseudomonas aeruginosa* : Souche 1 et Souches 4 à 11.
*Escherichia coli* : Souche 2 et Souches 12 à 20.
*Staphylococcus aureus* : Souche 3 et Souches 21 à 24.
Echantillons inconnus : Liquide 1 à Liquides 15

Des courbes de corrélation des peptides sont réalisées pour les différentes souches d'une même espèce, de manière à déterminer les peptides conservant le même comportement quelle que soit la souche utilisée. Ces courbes sont obtenues avec les peptides présentés dans le TABLEAU 2 *supra.*

Les figures 5, 6 et 7 illustrent pour chacune des espèces, la corrélation entre la concentration de bactéries présentes dans l'échantillon et la quantité de peptide mesurée par spectrométrie de masse MRM. Cette corrélation est mesurée entre au moins cinq souches différentes par espèce. Les coefficients de corrélation et les équations des droites de calibration correspondantes figurent dans le **TABLEAU 5** ci-dessous. Dans l'équation, x est la quantité de peptides mesurée par MRM en réalisant le ratio de l'aire sous pic chromatographique du peptide naturel divisé par l'aire sous le pic chromatographique du peptide lourd ; Y est la quantité de bactéries calculée en CFU/mL ; ln(x) représente le logarithme népérien de x.

**TABLEAU 5**

| **Espèce** | **Protéine** | **Peptide** | **R²** | **Equation** |
|---|---|---|---|---|
| *P.aeruginosa* | A3LLD0 | ADAAANDTLK | 0.96 | Y=e^{(1.04ln(x)+21.1)} |
| *E.coli* | ADHE | DYVEGETAAK | 0.97 | Y=e^{(0.88ln(x)+17.1)} |
| *S.aureus* | GUAA | LGIELGIPEHLVWR | 0.96 | Y=e^{(1.08ln(x)+21.5)} |

Les deux exemples précédents nous permettent de valider l'utilisation de ces peptides pour la quantification de bactéries par spectrométrie de masse MRM, en réalisant une quantification par étalonnage externe avec une gamme de souche pure.

Ces exemples nous permettent aussi de valider que les peptides choisis sont représentatifs de la quantité de bactéries présente dans un échantillon.

L'équation calculée pour la droite d'étalonnage externe permet ainsi de calculer en CFU/mL la quantité de bactéries présentes dans un échantillon inconnu. Les quantités de bactéries sont reprises dans le **TABLEAU 6** ci-dessous.

**TABLEAU 6**

| **Echantillon** | **Concentration de *P. aeruginosa* (CFU/ml)** | **Concentration d'*E. coli* (CFU/ml)** | **Concentration de *S. aureus* (CFU/ml)** |
|---|---|---|---|
| Liquide 1 | 7.18 10⁷ | 0 | 0 |
| Liquide 2 | 1.64 10⁷ | 0 | 0 |
| Liquide 3 | 0 | 0 | 2.7 10⁷ |
| Liquide 4 | 0 | 0 | 4.0 10⁸ |
| Liquide 5 | 1.00 10⁹ | 0 | 0 |
| Liquide 6 | 0 | 0 | 1.0 10⁹ |
| Liquide 7 | 0 | 0 | 5.0 10⁸ |
| Liquide 8 | 0 | 0 | 3.5 10⁸ |
| Liquide 9 | 1.39 10⁸ | 0 | 0 |
| Liquide 10 | 0 | 0 | 3.0 10⁸ |
| Liquide 11 | 1.23 10⁸ | 0 | 0 |
| Liquide 12 | 0 | 0 | 4.2 10⁸ |
| Liquide 13 | 0 | 0 | 3.0 10⁷ |
| Liquide 14 | 0 | 0 | 5.0 10⁸ |
| Liquide 15 | 0 | 0 | 0 |

De façon particulièrement avantageuse, le procédé ainsi développé permet de quantifier au moins trois espèces bactériennes dans un échantillon inconnu.

### Exemple 2 : Mesure du taux d'expression de l'enzyme VanA

### 1. Mise en culture de l'échantillon sur milieu de culture en absence d'antibiotique :

Les milieux de culture optimaux et les conditions de culture optimales sont différents selon les espèces de microorganisme. Par défaut l'échantillon est ensemencé sur différents milieux :
∘ gélose Columbia au sang de mouton (référence bioMérieux 43041) pendant 18 à 24 h à 35°C, en atmosphère aérobie ou anaérobie ;
∘ gélose TSA (référence bioMérieux 43011) pendant 18 à 24 h à 37°C.

### 2. Obtention de protéines digérées à partir de microorganismes

Le protocole suivant est mis en œuvre en 17 étapes :
a) Prélèvement d'une colonie de microorganisme, obtenue selon l'exemple 2 ou 3, ou d'un échantillon enrichi selon l'exemple 1, et mise en suspension dans 10 à 100µl d'une solution d'hydrochlorure de guanidine 6M, 50 mM Tris-HCl, pH=8,0.
b) Ajout de dithiothréitol (DTT) pour obtenir une concentration finale de 5mM.
c) Réduction pendant 20 minutes à 95°C dans un bain-marie.
d) Refroidissement des tubes à température ambiante.
e) Ajout d'iodoacétamide pour obtenir une concentration finale de 12,5 mM.
f) Alkylation pendant 40 minutes à température ambiante et à l'obscurité.
g) Dilution d'un facteur 6 avec une solution de NH₄HCO₃ 50 mM, pH=8.0 pour obtenir une concentration finale en hydrochlorure de guanidine de 1M.
h) Ajout de 1 µg de trypsine.
i) Digestion à 37°C pendant 6 heures jusqu'à une nuit.
j) Ajout d'acide formique à 0,5% jusqu'à un pH inférieur à 4 pour stopper la réaction.
k) Le volume d'échantillon est complété à 1mL avec eau/acide formique 0,5% (v/v)
l) Equilibrage des colonnes HLB Oasis Waters avec 1ml de méthanol puis 1 ml H₂O/acide formique 0,1% (v/v)
m) Dépôt de l'échantillon qui s'écoule par gravité
n) Lavage avec 1 mL H₂O/acide formique 0,1% (v/v)
o) Elution avec 1mL d'un mélange 80% de méthanol et 20% d'eau/acide formique 0,1% (v/v)
p) L'éluat est évaporé avec un évaporateur de type SpeedVac^{®} SPD2010 (Thermo Electron Corporation, Waltham, Massachusetts, Etats-Unis d'Amérique), durant 2 heures, afin d'obtenir un volume d'environ 100µl.
q) L'éluat est ensuite repris dans une solution eau/acide formique 0,5% (v/v) quantité suffisante pour (QSP) 200µl.

### 3. Quantification du taux d'expression de l'enzyme VanA

L'enzyme VanA confère la résistance à la Vancomycine chez les entérocoques. Le taux d'expression de cet enzyme peut être induit par la présence de la Vancomycine. Sans induction le taux d'expression est également très variable selon les souches bactériennes. Pour mesurer le taux d'expression de VanA, il faut mesurer la quantité de VanA et la quantité de bactéries présentes dans l'échantillon. Le ratio entre ces deux quantités permettra de mesurer le taux d'expression de la protéine VanA. La quantité de VanA présente dans les échantillons est mesurée en utilisant des peptides décrits dans la demande de brevet WO-A-2014/020276. La quantité de bactéries présentes dans les échantillons est mesurée en utilisant des peptides issus des 3 protéines RL29, RS19 et RL22.

L'enzyme Van A est quantifiée dans 3 échantillons d'*Enterococcus faecalis* (Ech1 à Ech3), obtenus à partir de cultures pures, selon la méthode suivante :
La colonie de microorganismes est obtenue conformément au paragraphe a), traitée selon le paragraphe b), puis un volume de 50µl de protéines digérées est injecté et analysé selon les conditions suivantes :
- Chaîne chromatographique Dionex Ultimate 3000 de la société Dionex Corporation (Sunnyvale, Etats Unis d'Amérique).
- Colonne Waters BEH130 C18, 2,1mm de diamètre interne, 100mm de long, taille de particule de 3,5µm (Waters, Saint-Quentin en Yvelines, France).
- Solvant A : H₂O + 0,1% acide formique.
- Solvant B : ACN + 0,1% acide formique.

Gradient HPLC défini dans le **TABLEAU 7** ci-après.

**TABLEAU 7**

| **Temps (min)** | **Débit (µl)** | **Solvant A (%)** | **Solvant B (%)** |
|---|---|---|---|
| 0 | 300 | 98 | 2 |
| 3 | 300 | 98 | 2 |
| 28 | 300 | 63 | 37 |
| 30 | 300 | 0 | 100 |
| 38 | 300 | 0 | 100 |
| 38.1 | 300 | 98 | 2 |
| 45 | 300 | 98 | 2 |

L'éluat en sortie de colonne chromatographique est directement injecté dans la source d'ionisation du spectromètre de masse de type QTRAP^{®} 5500 de la société

AB Sciex (Framingham, Massachusetts,, Etats Unis d'Amérique).
- Les peptides, issus de la digestion des protéines du microorganisme, sont analysés par le spectromètre de masse en mode MRM. Seuls les peptides indiqués dans le **TABLEAU 8** sont détectés. Pour cela, le ou les fragments indiqué(s) dans le **TABLEAU 8** sont détecté(s).

**TABLEAU 8**

| **Transition numéro** | **Protéine** | **Peptide** | **Etat de charge du précurseur** | **Ion fragment de première génération** |
|---|---|---|---|---|
| 1 | VanA | IHQEVEPEK | 2 | y5 monochargé |
| 2 | VanA | IHQEVEPEK | 2 | y6 monochargé |
| 3 | VanA | IHQEVEPEK | 2 | y7 monochargé |
| 4 | VanA | LIVLALK | 2 | y4 monochargé |
| 5 | VanA | LIVLALK | 2 | y5 monochargé |
| 6 | VanA | LIVLALK | 2 | y6 monochargé |
| 7 | VanA | LQYGIFR | 2 | y4 monochargé |
| 8 | VanA | LQYGIFR | 2 | y5 monochargé |
| 9 | VanA | LQYGIFR | 2 | y6 monochargé |
| 10 | VanA | MHGLLVK | 2 | b5 monochargé |
| 11 | VanA | MHGLLVK | 2 | y5 monochargé |
| 12 | VanA | MHGLLVK | 2 | y6 monochargé |
| 13 | VanA | MMAAAGIALPELIDR | 2 | y6 monochargé |
| 14 | VanA | MMAAAGIALPELIDR | 2 | y7 monochargé |
| 15 | VanA | MMAAAGIALPELIDR | 2 | y8 monochargé |
| 16 | VanA | NAGIATPAFWVINK | 2 | y10 monochargé |
| 17 | VanA | NAGIATPAFWVINK | 2 | y8 monochargé |
| 18 | VanA | NAGIATPAFWVINK | 2 | y9 monochargé |
| 19 | VanA | SAIEIAANINK | 2 | y6 monochargé |
| 20 | VanA | SAIEIAANINK | 2 | y7 monochargé |
| 21 | VanA | SAIEIAANINK | 2 | y8 monochargé |
| 22 | VanA | SGSSFGVK | 2 | y5 monochargé |
| 23 | VanA | SGSSFGVK | 2 | y6 monochargé |
| 24 | VanA | SGSSFGVK | 2 | y7 monochargé |
| 25 | VanA | SLTYIVAK | 2 | y4 monochargé |
| 26 | VanA | SLTYIVAK | 2 | y5 monochargé |
| 27 | VanA | SLTYIVAK | 2 | y6 monochargé |
| 28 | VanA | VDMFLQDNGR | 2 | y6 monochargé |
| 29 | VanA | VDMFLQDNGR | 2 | y7 monochargé |
| 30 | VanA | VDMFLQDNGR | 2 | y8 monochargé |
| 31 | VanA | VNSADELDYAIESAR | 2 | y6 monochargé |
| 32 | VanA | VNSADELDYAIESAR | 2 | y7 monochargé |
| 33 | VanA | VNSADELDYAIESAR | 2 | y8 monochargé |
| 34 | VanA | YEPLYIGITK | 2 | y7 monochargé |
| 35 | VanA | YEPLYIGITK | 2 | y8 monochargé |
| 36 | VanA | YEPLYIGITK | 2 | y8 dichargé |
| 37 | RL22 | LVIDLIR | 2 | y4 monochargé |
| 38 | RL22 | LVIDLIR | 2 | y5 monochargé |
| 39 | RL22 | LVIDLIR | 2 | y6 monochargé |
| 40 | RL29 | FQLATGQLENTAR | 2 | y10 monochargé |
| 41 | RL29 | FQLATGQLENTAR | 2 | y8 monochargé |
| 42 | RL29 | FQLATGQLENTAR | 2 | y9 monochargé |
| 43 | RS19 | LGEFAPTR | 2 | y5 monochargé |
| 44 | RS19 | LGEFAPTR | 2 | y6 monochargé |
| 45 | RS19 | LGEFAPTR | 2 | y7 monochargé |

Les transitions mentionnées dans le **TABLEAU 8** sont détectées en utilisant les paramètres figurant dans les **TABLEAUX 9** et **10,** ci-dessous.

**TABLEAU 9**

| **Transition numéro** | **Temps de rétention** | **(m/z) filtré en Q1** | **(m/z) filtré en Q3** | **Energie de collision (eV)** |
|---|---|---|---|---|
| 1 | 8,75 | 554,79 | 601,32 | 29 |
| 2 | 8,75 | 554,79 | 730,36 | 29 |
| 3 | 8,75 | 554,79 | 858,42 | 29 |
| 4 | 19,71 | 385,28 | 444,32 | 22 |
| 5 | 19,71 | 385,28 | 543,39 | 22 |
| 6 | 19,71 | 385,28 | 656,47 | 22 |
| 7 | 17,91 | 448,75 | 492,29 | 25 |
| 8 | 17,91 | 448,75 | 655,36 | 25 |
| 9 | 17,91 | 448,75 | 783,41 | 25 |
| 10 | 13,32 | 399,24 | 552,3 | 23 |
| 11 | 13,32 | 399,24 | 529,37 | 23 |
| 12 | 13,32 | 399,24 | 666,43 | 23 |
| 13 | 23,29 | 786,42 | 742,41 | 32 |
| 14 | 23,29 | 786,42 | 855,49 | 31 |
| 15 | 23,29 | 786,42 | 926,53 | 34 |
| 16 | 22,44 | 751,41 | 1146,63 | 38 |
| 17 | 22,44 | 751,41 | 974,55 | 38 |
| 18 | 22,44 | 751,41 | 1075,59 | 38 |
| 19 | 15,89 | 572,32 | 630,36 | 25 |
| 20 | 15,89 | 572,32 | 743,44 | 26 |
| 21 | 15,89 | 572,32 | 872,48 | 25 |
| 22 | 10,67 | 384,7 | 537,3 | 20,5 |
| 23 | 10,67 | 384,7 | 624,34 | 18,5 |
| 24 | 10,67 | 384,7 | 681,36 | 19,5 |
| 25 | 15,29 | 447,77 | 430,3 | 25 |
| 26 | 15,29 | 447,77 | 593,37 | 25 |
| 27 | 15,29 | 447,77 | 694,41 | 25 |
| 28 | 16,64 | 597,78 | 702,35 | 29 |
| 29 | 16,64 | 597,78 | 849,42 | 29 |
| 30 | 16,64 | 597,78 | 980,46 | 27 |
| 31 | 18,52 | 826,89 | 646,35 | 41 |
| 32 | 18,52 | 826,89 | 809,42 | 41 |
| 33 | 18,52 | 826,89 | 924,44 | 41 |
| 34 | 19,2 | 598,83 | 807,5 | 31 |
| 35 | 19,2 | 598,83 | 904,55 | 22 |
| 36 | 19,2 | 598,83 | 452,78 | 25 |
| 37 | **20.55** | 421.28 | 516.31 | 24 |
| 38 | 20.55 | 421.28 | 629.4 | 24 |
| 39 | 20.55 | 421.28 | 728.47 | 24 |
| 40 | 16.75 | 724.88 | 1060.54 | 37 |
| 41 | 16.75 | 724.88 | 888.45 | 37 |
| 42 | 16.75 | 724.88 | 989.5 | 37 |
| 43 | 13.45 | 445.74 | 591.32 | 25 |
| 44 | 13.45 | 445.74 | 720.37 | 25 |
| 45 | 13.45 | 445.74 | 777.39 | 25 |

**TABLEAU 10**

| **Transition numéro** | **Potentiel d'orifice** | **potentiel d'entrée avant Q0** | **Potentiel en sortie de cellule de collision** | **Seuil de positivité** |
|---|---|---|---|---|
| 1 | 80 | 10 | 35 | 2000 |
| 2 | 80 | 10 | 35 | 2000 |
| 3 | 80 | 10 | 35 | 2000 |
| 4 | 80 | 10 | 35 | 1400 |
| 5 | 80 | 10 | 35 | 2000 |
| 6 | 80 | 10 | 35 | 2000 |
| 7 | 80 | 10 | 35 | 2000 |
| 8 | 80 | 10 | 35 | 2000 |
| 9 | 80 | 10 | 35 | 1500 |
| 10 | 80 | 10 | 35 | 4000 |
| 11 | 80 | 10 | 35 | 4000 |
| 12 | 80 | 10 | 35 | 4000 |
| 13 | 120 | 10 | 18 | 2000 |
| 14 | 120 | 10 | 18 | 2000 |
| 15 | 120 | 10 | 18 | 2000 |
| 16 | 80 | 10 | 35 | 2000 |
| 17 | 80 | 10 | 35 | 2000 |
| 18 | 80 | 10 | 35 | 2000 |
| 19 | 90 | 10 | 15 | 2000 |
| 20 | 90 | 10 | 15 | 2000 |
| 21 | 90 | 10 | 15 | 2000 |
| 22 | 85 | 10 | 24 | 2000 |
| 23 | 85 | 10 | 24 | 2000 |
| 24 | 85 | 10 | 24 | 2000 |
| 25 | 80 | 10 | 35 | 2000 |
| 26 | 80 | 10 | 35 | 1500 |
| 27 | 80 | 10 | 35 | 2000 |
| 28 | 100 | 10 | 17 | 2000 |
| 29 | 100 | 10 | 17 | 2000 |
| 30 | 100 | 10 | 17 | 2000 |
| 31 | 80 | 10 | 35 | 1000 |
| 32 | 80 | 10 | 35 | 2000 |
| 33 | 80 | 10 | 35 | 2000 |
| 34 | 105 | 10 | 20 | 1400 |
| 35 | 105 | 10 | 20 | 2000 |
| 36 | 105 | 10 | 20 | 2000 |
| 37 | 100 | 4 | 35 | 2000 |
| 38 | 100 | 4 | 35 | 2000 |
| 39 | 100 | 4 | 35 | 2000 |
| 40 | 100 | 4 | 35 | 2000 |
| 41 | 100 | 4 | 35 | 2000 |
| 42 | 100 | 4 | 35 | 2000 |
| 43 | 100 | 4 | 35 | 2000 |
| 44 | 100 | 4 | 35 | 2000 |
| 45 | 100 | 4 | 35 | 2000 |

- Les autres paramètres machine utilisés sont les suivants :

| | |
|---|---|
| Type de balayage : | MRM |
| MRM planifié : | oui |
| Polarité : | Positive |
| Source d'ionisation : | Turbo V^{™} (AB Sciex) |
| Réglage Q1 : | Filtrage avec résolution unitaire |
| Réglage Q3 : | Filtrage avec résolution unitaire |
| Pause inter-scan : | 5.00 msec |
| Vitesse de balayage : | 10 Da/s |

| | |
|---|---|
| Gaz rideau : | 50,00 psi |
| Tension de cône : | 5500,00 V |
| Température de source : | 550,00 °C |
| Gaz de nébulisation : | 50,00 psi |
| Gaz chauffant : | 40,00 psi |
| Gaz de collision induisant dissociation : | 9,00 psi |
| Remplissage dynamique : | inactivé |
| Temps de cycle total : | 1.2 sec |
| Fenêtre de détection : | 90 sec |

Les aires obtenues pour chacune des transitions et pour chacun des microorganismes étudiés ont été mesurées. Lorsque l'aire d'une transition est supérieure ou égale au seuil de positivité décrit dans le **TABLEAU 10,** la détection de la transition est considérée comme positive et son signal en unité arbitraire est reporté dans **TABLEAU 11.** Lorsque l'aire d'une transition est inférieure au seuil de positivité décrit dans le **TABLEAU 10,** la détection de la transition est considérée comme négative et est notée « 0 » dans le **TABLEAU 11.**

**TABLEAU 11**

| **Transition numéro** | **Ech1** | **Ech2** | **Ech3** | **Ech4** | **Ech5** | **Ech6** | **Ech7** | **Ech8** | **Ech9** | **Ech10** | **Ech11** |
|---|---|---|---|---|---|---|---|---|---|---|---|
| 1 | 0 | 0 | 0 | 0 | 0 | 0 | 0 | 0 | 0 | 0 | 0 |
| 2 | 0 | 0 | 0 | 0 | 0 | 0 | 0 | 0 | 0 | 0 | 0 |
| 3 | 0 | 0 | 0 | 0 | 0 | 0 | 0 | 0 | 0 | 0 | 0 |
| 4 | 0 | 0 | 0 | 0 | 0 | 0 | 0 | 0 | 0 | 1463 | 0 |
| 5 | 0 | 0 | 0 | 0 | 0 | 0 | 0 | 0 | 0 | 13020 | 0 |
| 6 | 2915 | 0 | 5471 | 0 | 0 | 0 | 0 | 0 | 0 | 2647 | 0 |
| 7 | 0 | 0 | 0 | 0 | 0 | 0 | 0 | 0 | 0 | 2836 | 0 |
| 8 | 0 | 11040 | 0 | 0 | 0 | 0 | 0 | 0 | 0 | 10590 | 0 |
| 9 | 0 | 6243 | 0 | 0 | 0 | 0 | 0 | 0 | 0 | 1869 | 0 |
| 10 | 0 | 0 | 0 | 0 | 0 | 0 | 0 | 0 | 0 | 8169 | 0 |
| 11 | 0 | 0 | 8101 | 4026 | 0 | 4572 | 4169 | 0 | 0 | 25590 | 0 |
| 12 | 0 | 0 | 0 | 0 | 0 | 0 | 0 | 0 | 0 | 8767 | 0 |
| 13 | 0 | 2090 | 0 | 0 | 0 | 0 | 0 | 0 | 0 | 0 | 0 |
| 14 | 0 | 0 | 0 | 0 | 0 | 0 | 0 | 0 | 0 | 0 | 0 |
| 15 | 0 | 0 | 0 | 0 | 0 | 0 | 0 | 0 | 0 | 0 | 0 |
| 16 | 0 | 0 | 0 | 0 | 0 | 0 | 0 | 0 | 0 | 0 | 0 |
| 17 | 0 | 0 | 0 | 0 | 0 | 0 | 0 | 0 | 0 | 0 | 0 |
| 18 | 0 | 0 | 0 | 0 | 0 | 0 | 0 | 0 | 0 | 0 | 0 |
| 19 | 0 | 0 | 9028 | 0 | 0 | 0 | 0 | 0 | 0 | 389800 | 5610 |
| 20 | 0 | 0 | 6689 | 0 | 0 | 0 | 0 | 0 | 0 | 274700 | 4391 |
| 21 | 0 | 11610 | 7003 | 0 | 0 | 0 | 0 | 0 | 0 | 245300 | 4541 |
| 22 | 3069 | 0 | 18170 | 0 | 0 | 0 | 11340 | 0 | 0 | 53270 | 9942 |
| 23 | 0 | 0 | 7476 | 0 | 0 | 0 | 0 | 0 | 0 | 153400 | 5372 |
| 24 | 0 | 0 | 3490 | 0 | 0 | 0 | 0 | 0 | 0 | 68260 | 2667 |
| 25 | 2782 | 2536 | 4446 | 3109 | 3545 | 0 | 4009 | 2208 | 3027 | 3292 | 4181 |
| 26 | 0 | 2421 | 0 | 0 | 0 | 0 | 0 | 0 | 0 | 1625 | 0 |
| 27 | 0 | 0 | 2153 | 0 | 0 | 0 | 0 | 0 | 0 | 5453 | 0 |
| 28 | 0 | 0 | 0 | 0 | 0 | 0 | 0 | 0 | 0 | 2409 | 0 |
| 29 | 0 | 0 | 0 | 0 | 0 | 0 | 0 | 0 | 0 | 0 | 0 |
| 30 | 0 | 0 | 0 | 0 | 0 | 0 | 0 | 0 | 0 | 0 | 0 |
| 31 | 0 | 0 | 0 | 0 | 0 | 0 | 0 | 0 | 0 | 1028 | 0 |
| 32 | 0 | 2210 | 0 | 0 | 0 | 0 | 0 | 0 | 0 | 2794 | 0 |
| 33 | 0 | 0 | 0 | 0 | 0 | 0 | 0 | 0 | 0 | 2540 | 0 |
| 34 | 0 | 0 | 2089 | 0 | 0 | 2317 | 0 | 0 | 0 | 33950 | 1416 |
| 35 | 0 | 0 | 7596 | 0 | 0 | 0 | 0 | 0 | 0 | 179600 | 5836 |
| 36 | 2158 | 0 | 13690 | 0 | 0 | 0 | 0 | 0 | 0 | 290000 | 9278 |
| 37 | 1623000 | 4407000 | 3504000 | 694600 | 1063000 | 3283000 | 1951000 | 774400 | 294600 | 935300 | 949800 |
| 38 | 5766000 | 14880000 | 12010000 | 2500000 | 3820000 | 10980000 | 6640000 | 2710000 | 1067000 | 3290000 | 3417000 |
| 39 | 690300 | 1884000 | 1437000 | 306600 | 462500 | 1389000 | 813000 | 317900 | 124500 | 393800 | 398200 |
| 40 | 525500 | 624600 | 588800 | 236800 | 297600 | 430300 | 326400 | 117700 | 139800 | 210800 | 170400 |
| 41 | 1005000 | 1169000 | 1122000 | 448000 | 569300 | 810600 | 626100 | 225900 | 260300 | 417500 | 321500 |
| 42 | 749600 | 926300 | 861500 | 336700 | 436500 | 635000 | 481100 | 175700 | 206800 | 316100 | 256300 |
| 43 | 3261000 | 3658000 | 3390000 | 1286000 | 1685000 | 3011000 | 2298000 | 954400 | 607700 | 1409000 | 1154000 |
| 44 | 1239000 | 1442000 | 1346000 | 497800 | 680500 | 1194000 | 900600 | 346300 | 232900 | 535000 | 462900 |
| 45 | 3836000 | 4295000 | 4080000 | 1542000 | 2065000 | 3715000 | 2668000 | 1079000 | 727500 | 1616000 | 1400000 |

Lorsque les trois transitions d'un même peptide sont notées supérieures à « 0 », la détection du peptide est considérée comme positive. L'aire de l'ensemble des transitions des peptides positifs VanA est sommée pour mesurer la quantité de protéine VanA. De même, l'ensemble des aires des peptides des protéines RL22, RL29 et RS19 est sommé pour mesurer la quantité de bactéries. Le ratio de la somme des aires des peptides VanA sur la somme des aires des peptides des protéines RL22, RL29 et RS19 permet de mesurer le taux d'expression relatif de la protéine VanA dans la souche bactérienne de l'échantillon testé. Ce taux d'expression relatif peut également être exprimé en pourcentage du taux d'expression de l'échantillon ayant le plus fort taux d'expression relatif de VanA, ici l'échantillon Ech10.

**TABLEAU 12**

| **Echantillon** | **Quantité de VanA (unité arbitraire)** | **Quantité de bactéries (unité arbitraire)** | **Taux d'expression relatif de VanA** | **Taux d'expression de VanA exprimé en pourcentage du taux d'expression de l'échantillon Ech10** |
|---|---|---|---|---|
| Ech1 | 0 | 18695400 | 0 | 0 |
| Ech2 | 0 | 33285900 | 0 | 0 |
| Ech3 | 75231 | 28339300 | 0,002654653 | 1,43 |
| Ech4 | 0 | 7848500 | 0 | 0 |
| Ech5 | 0 | 11079400 | 0 | 0 |
| Ech6 | 0 | 25447900 | 0 | 0 |
| Ech7 | 0 | 16704200 | 0 | 0 |
| Ech8 | 0 | 6701300 | 0 | 0 |
| Ech9 | 0 | 3661100 | 0 | 0 |
| Ech10 | 1688280 | 9123500 | 0,185047405 | 100 |
| Ech11 | 49053 | 8530100 | 0,005750577 | 3,11 |

De façon particulièrement avantageuse, les quantités de VanA et de bactéries ont été mesurées de façon simultanée à l'aide de la même méthode MRM et nous avons ainsi pu mesurer en une seule analyse le taux d'expression relatif de VanA. Il est ainsi possible de constater très facilement que l'échantillon Ech10 à un taux d'expression très élevé de VanA par rapport aux autres échantillons, que les échantillons Ech3 et Ech11 ont un taux d'expression faible et tous les autres échantillons ont un taux d'expression nul.

### Exemple 3 : Mesure du taux d'expression de porines chez Klebsiella pneumoniae

### 1. Mise en culture de l'échantillon

Les milieux de culture optimaux et les conditions de culture optimales sont différents selon les espèces de microorganisme. Par défaut, l'échantillon est ensemencé sur gélose Columbia au sang de mouton (référence bioMérieux 43041) pendant 18 à 24 h à 35°C, en atmosphère aérobie.

### 2. Obtention de protéines digérées à partir de microorganismes

Le protocole suivant est mis en œuvre en 17 étapes :
a) Prélèvement d'une colonie de microorganisme, obtenue selon l'exemple 2 ou 3, ou d'un échantillon enrichi selon l'exemple 1, et mise en suspension dans 10 à 100µl d'une solution d'hydrochlorure de guanidine 6M, 50 mM Tris-HCl, pH=8,0.
b) Ajout de dithiothréitol (DTT) pour obtenir une concentration finale de 5mM.
c) Réduction pendant 20 minutes à 95°C dans un bain-marie.
d) Refroidissement des tubes à température ambiante.
e) Ajout d'iodoacétamide pour obtenir une concentration finale de 12,5 mM.
f) Alkylation pendant 40 minutes à température ambiante et à l'obscurité.
g) Dilution d'un facteur 6 avec une solution de NH₄HCO₃ 50 mM, pH=8.0 pour obtenir une concentration finale en hydrochlorure de guanidine de 1M.
h) Ajout de 1 µg de trypsine.
i) Digestion à 37°C pendant 6 heures jusqu'à une nuit.
j) Ajout d'acide formique à 0,5% jusqu'à un pH inférieur à 4 pour stopper la réaction.
k) Le volume d'échantillon est complété à 1mL avec eau/acide formique 0,5% (v/v)
l) Equilibrage des colonnes HLB Oasis Waters avec 1ml de méthanol puis 1 ml H₂O/acide formique 0,1% (v/v)
m) Dépôt de l'échantillon qui s'écoule par gravité
n) Lavage avec 1 mL H2O/acide formique 0,1% (v/v)
o) Elution avec 1mL d'un mélange 80% de méthanol et 20% d'eau/acide formique 0,1% (v/v)
p) L'éluat est évaporé avec un évaporateur de type SpeedVac^{®} SPD2010 (Thermo Electron Corporation, Waltham, Massachusetts, Etats-Unis d'Amérique), durant 2 heures, afin d'obtenir un volume d'environ 100µl.
q) L'éluat est ensuite repris dans une solution eau/acide formique 0,5% (v/v) quantité suffisante pour (QSP) 200µl.

### 4. Quantification du taux d'expression des porines

Les porines sont des protéines de la paroi bactérienne permettant la diffusion de molécules à travers cette membrane. Elles peuvent être impliquées ou non dans la résistance. Ainsi pour les premières, la diminution de leur quantité implique une diminution de la sensibilité de la bactérie, souvent à plusieurs antibiotiques. Il est donc important de quantifier les porines liées à la résistance dans les souches.

Dans cet exemple, les porines recherchées sont les suivantes : LamB, OmpA, OmpF. Le rôle d'OmpF dans le développement de résistance aux antibiotiques est établi. Les autres porines ne sont, a priori, pas impliquées dans la résistance [37].

Les porines seront quantifiées dans les 14 souches numérotées de Ech1 à Ech14

La colonie de microorganismes est obtenue conformément au paragraphe a), traitée selon le paragraphe b), puis un volume de 50µl de protéines digérées est injecté et analysé selon les conditions suivantes :
- Chaîne chromatographique Nexera de la société SHIMADZU (Kyoto, Japon).
- Colonne Waters (Waters, Saint-Quentin en Yvelines, France) BEH C18, 2,1mm de diamètre interne, 100mm de long, taille de particule de 3,5µm.
- Solvant A : H₂O + 0,1% acide formique.
- Solvant B : ACN + 0,1% acide formique.

Le Gradient HPLC défini dans le **TABLEAU 13** ci-après.

**TABLEAU 13**

| **Temps (min)** | **Débit (µl)** | **Solvant A (%)** | **Solvant B (%)** |
|---|---|---|---|
| 0 | 300 | 95 | 5 |
| 3 | 300 | 95 | 5 |
| 26 | 300 | 65 | 35 |
| 26.1 | 300 | 5 | 95 |
| 28 | 300 | 5 | 95 |

L'éluat en sortie de colonne chromatographique est directement injecté dans la source d'ionisation du spectromètre de masse de type QTRAP^{®} 5500 de la société AB Sciex (Framingham, Massachusetts, Etats Unis d'Amérique).

Les transitions utilisées pour la quantification des bactéries sont décrites dans le **TABLEAU 14.**

**TABLEAU 14**

| **Transition numéro** | **(m/z) filtré en Q1** | **(m/z) filtré en Q3** | **Protéine** | **Séquence peptidique** | **Etat de charge precurseur** | **Ion fragment** |
|---|---|---|---|---|---|---|
| 1 | 547.95 | 766.432 | SEQ ID N° 15 | AFAASGELEVNHLQR | 3 | y6 monochargé |
| 2 | 547.95 | 667.363 | SEQ ID N° 15 | AFAASGELEVNHLQR | 3 | y5 monochargé |
| 3 | 547.95 | 712.368 | SEQ ID N° 15 | AFAASGELEVNHLQR | 3 | y13 dichargé |
| 4 | 642.874 | 1058.573 | SEQ ID N° 17 | LLVSELTGVEPK | 2 | y10 monochargé |
| 5 | 642.874 | 959.504 | SEQ ID N° 17 | LLVSELTGVEPK | 2 | y9 monochargé |
| 6 | 642.874 | 743.43 | SEQ ID N° 17 | LLVSELTGVEPK | 2 | y7 monochargé |
| 7 | 551.791 | 902.458 | SEQ ID N° 19 | SLQAQVAEEK | 2 | y8 monochargé |
| 8 | 551.791 | 774.399 | SEQ ID N° 19 | SLQAQVAEEK | 2 | y7 monochargé |
| 9 | 551.791 | 703.362 | SEQ ID N° 19 | SLQAQVAEEK | 2 | y6 monochargé |

Les transitions utilisées pour quantifier l'expression des porines sont décrites dans le **TABLEAU 15.** Les séquences de LamB, OmpA, OmpF (OmpK35) correspondent respectivement à la SEQ ID NO 21, SEQ ID NO 27, SEQ ID NO 32.

**TABLEAU 15**

| **Numero transition** | **(m/z) filtré en Q1** | **(m/z) filtré en Q3** | **Proteine** | **Sequence peptidique** | **Etat de charge du precurseur** | **Fragment premiere génération** |
|---|---|---|---|---|---|---|
| 10 | 579.755 | 450.231 | LamB | DSSGSGAFTSSR | 2 | y4 |
| 11 | 579.755 | 725.358 | LamB | DSSGSGAFTSSR | 2 | y7 |
| 12 | 579.755 | 812.39 | LamB | DSSGSGAFTSSR | 2 | y8 |
| 13 | 579.755 | 869.411 | LamB | DSSGSGAFTSSR | 2 | y9 |
| 14 | 407.229 | 218.15 | LamB | IFATYAK | 2 | y2 |
| 15 | 407.229 | 381.213 | LamB | IFATYAK | 2 | y3 |
| 16 | 407.229 | 482.261 | LamB | IFATYAK | 2 | y4 |
| 17 | 407.229 | 553.298 | LamB | IFATYAK | 2 | y5 |
| 18 | 677.357 | 395.204 | LamB | LAGLETNPGGVLELGVD YGR | 3 | y3 |
| 19 | 677.357 | 666.321 | LamB | LAGLETNPGGVLELGVD YGR | 3 | y6 |
| 20 | 677.357 | 779.405 | LamB | LAGLETNPGGVLELGVD YGR | 3 | y7 |
| 21 | 677.357 | 908.447 | LamB | LAGLETNPGGVLELGVD YGR | 3 | y8 |
| 22 | 437.245 | 333.192 | LamB | LGQELWK | 2 | y2 |
| 23 | 437.245 | 575.319 | LamB | LGQELWK | 2 | y4 |
| 24 | 437.245 | 703.377 | LamB | LGQELWK | 2 | y5 |
| 25 | 437.245 | 760.399 | LamB | LGQELWK | 2 | y6 |
| 26 | 841.345 | 565.246 | LamB | NSESGGSYTFSSDDTK | 2 | y5 |
| 27 | 841.345 | 652.278 | LamB | NSESGGSYTFSSDDTK | 2 | y6 |
| 28 | 841.345 | 1063.458 | LamB | NSESGGSYTFSSDDTK | 2 | y9 |
| 29 | 596.832 | 708.393 | OmpA | AQSVVDYLVAK | 2 | y6 |
| 30 | 596.832 | 807.461 | OmpA | AQSVVDYLVAK | 2 | y7 |
| 31 | 596.832 | 906.529 | OmpA | AQSVVDYLVAK | 2 | y8 |
| 32 | 596.832 | 993.562 | OmpA | AQSVVDYLVAK | 2 | y9 |
| 33 | 626.812 | 611.278 | OmpA | DGSAVVLGYTDR | 2 | y5 |
| 34 | 626.812 | 724.362 | OmpA | DGSAVVLGYTDR | 2 | y6 |
| 35 | 626.812 | 823.431 | OmpA | DGSAVVLGYTDR | 2 | y7 |
| 36 | 626.812 | 922.499 | OmpA | DGSAVVLGYTDR | 2 | y8 |
| 37 | 409.721 | 361.198 | OmpA | LGGMVWR | 2 | y2 |
| 38 | 409.721 | 460.267 | OmpA | LGGMVWR | 2 | y3 |
| 39 | 409.721 | 648.329 | OmpA | LGGMVWR | 2 | y5 |
| 40 | 542.277 | 522.267 | OmpA | SDVLFNFNK | 2 | y4 |
| 41 | 542.277 | 669.336 | OmpA | SDVLFNFNK | 2 | y5 |
| 42 | 542.277 | 782.42 | OmpA | SDVLFNFNK | 2 | y6 |
| 43 | 542.277 | 881.488 | OmpA | SDVLFNFNK | 2 | y7 |
| 44 | 611.262 | 395.204 | OmpF | AGEYGSFDYGR | 2 | y3 |
| 45 | 611.262 | 744.331 | OmpF | AGEYGSFDYGR | 2 | y6 |
| 46 | 611.262 | 801.353 | OmpF | AGEYGSFDYGR | 2 | y7 |
| 47 | 611.262 | 964.416 | OmpF | AGEYGSFDYGR | 2 | y8 |
| 48 | 568.783 | 717.378 | OmpF | AGFSGGDADLVK | 2 | y7 |
| 49 | 568.783 | 774.399 | OmpF | AGFSGGDADLVK | 2 | y8 |
| 50 | 568.783 | 861.431 | OmpF | AGFSGGDADLVK | 2 | y9 |
| 51 | 686.804 | 755.321 | OmpF | FNQLDDNDYTK | 2 | y6 |
| 52 | 686.804 | 870.348 | OmpF | FNQLDDNDYTK | 2 | y7 |
| 53 | 686.804 | 983.432 | OmpF | FNQLDDNDYTK | 2 | y8 |
| 54 | 441.227 | 510.267 | OmpF | TNGVATYR | 2 | y4 |
| 55 | 441.227 | 609.336 | OmpF | TNGVATYR | 2 | y5 |
| 56 | 441.227 | 666.357 | OmpF | TNGVATYR | 2 | y6 |

Les paramètres de la source d'ionisation sont décrites dans le **TABLEAU 16**

**TABLEAU 16**

| **Transition numéro** | **Potentiel d'orifice (V)** | **potentiel d'entrée avant Q0 (V)** | **Energie de collision (eV)** | **potentiel en sortie de cellule de collision (V)** |
|---|---|---|---|---|
| 1 | 71.1 | 10 | 27.4 | 9 |
| 2 | 71.1 | 10 | 27.4 | 9 |
| 3 | 71.1 | 10 | 27.4 | 9 |
| 4 | 78 | 10 | 32 | 9 |
| 5 | 78 | 10 | 32 | 9 |
| 6 | 78 | 10 | 32 | 9 |
| 7 | 71.3 | 10 | 28.7 | 9 |
| 8 | 71.3 | 10 | 28.7 | 9 |
| 9 | 71.3 | 10 | 28.7 | 9 |
| 10 | 73.4 | 10 | 29.7 | 9 |
| 11 | 73.4 | 10 | 29.7 | 9 |
| 12 | 73.4 | 10 | 29.7 | 9 |
| 13 | 73.4 | 10 | 29.7 | 9 |
| 14 | 60.8 | 10 | 23.5 | 9 |
| 15 | 60.8 | 10 | 23.5 | 9 |
| 16 | 60.8 | 10 | 23.5 | 9 |
| 17 | 60.8 | 10 | 23.5 | 9 |
| 18 | 80.5 | 10 | 34.4 | 9 |
| 19 | 80.5 | 10 | 34.4 | 9 |
| 20 | 80.5 | 10 | 34.4 | 9 |
| 21 | 80.5 | 10 | 34.4 | 9 |
| 22 | 63 | 10 | 24.6 | 9 |
| 23 | 63 | 10 | 24.6 | 9 |
| 24 | 63 | 10 | 24.6 | 9 |
| 25 | 63 | 10 | 24.6 | 9 |
| 26 | 92.5 | 10 | 39.1 | 9 |
| 27 | 92.5 | 10 | 39.1 | 9 |
| 28 | 92.5 | 10 | 39.1 | 9 |
| 29 | 74.6 | 10 | 30.3 | 9 |
| 30 | 74.6 | 10 | 30.3 | 9 |
| 31 | 74.6 | 10 | 30.3 | 9 |
| 32 | 74.6 | 10 | 30.3 | 9 |
| 33 | 76.8 | 10 | 31.4 | 9 |
| 34 | 76.8 | 10 | 31.4 | 9 |
| 35 | 76.8 | 10 | 31.4 | 9 |
| 36 | 76.8 | 10 | 31.4 | 9 |
| 37 | 61 | 10 | 23.6 | 9 |
| 38 | 61 | 10 | 23.6 | 9 |
| 39 | 61 | 10 | 23.6 | 9 |
| 40 | 70.6 | 10 | 28.4 | 9 |
| 41 | 70.6 | 10 | 28.4 | 9 |
| 42 | 70.6 | 10 | 28.4 | 9 |
| 43 | 70.6 | 10 | 28.4 | 9 |
| 44 | 75.7 | 10 | 30.9 | 9 |
| 45 | 75.7 | 10 | 30.9 | 9 |
| 46 | 75.7 | 10 | 30.9 | 9 |
| 47 | 75.7 | 10 | 30.9 | 9 |
| 48 | 72.6 | 10 | 29.3 | 9 |
| 49 | 72.6 | 10 | 29.3 | 9 |
| 50 | 72.6 | 10 | 29.3 | 9 |
| 51 | 81.2 | 10 | 33.6 | 9 |
| 52 | 81.2 | 10 | 33.6 | 9 |
| 53 | 81.2 | 10 | 33.6 | 9 |
| 54 | 63.3 | 10 | 24.7 | 9 |
| 55 | 63.3 | 10 | 24.7 | 9 |
| 56 | 63.3 | 10 | 24.7 | 9 |

Les autres paramètres du spectromètre utilisés sont les suivants :
Type de balayage : MRM
MRM planifié : oui
Polarité : Positive
Source d'ionisation : Turbo V^{™} (AB Sciex)
Réglage Q1 : Filtrage avec résolution unitaire
Réglage Q3 : Filtrage avec résolution unitaire
Pause inter-scan : 5.00 msec
Vitesse de balayage : 10 Da/s
Gaz rideau : 50,00 psi
Tension de cône : 5500,00 V
Température de source : 550,00 °C
Gaz de nébulisation : 50,00 psi
Gaz chauffant : 40,00 psi
Gaz de collision induisant dissociation : 9,00 psi
Remplissage dynamique : inactivé
Temps de cycle total : 1.2 sec
Fenêtre de détection : 90 sec

Les aires obtenues pour chacune des transitions, au temps de rétention spécifié et pour chacun des microorganismes étudiés ont été mesurées. Lorsque l'aire d'une transition est supérieure ou égale à 3000 unités arbitraires (u. a.), la détection de la transition est considérée comme positive et son signal en unité arbitraire est reporté dans **TABLEAU 17.** Lorsque l'aire d'une transition est inférieure à 3000 u. a. la détection de la transition est considérée comme négative et est notée « 0 » dans le **TABLEAU 17.**

**TABLEAU 17**

| Transitions Numero | Temps de retention (min) | **Ech1** | **Ech2** | **Ech3** | **Ech4** | **Ech5** | **Ech6** | **Ech7** | **Ech8** | **Ech9** | **Ech10** | **Ech11** | **Ech12** | **Ech13** | **Ech14** |
|---|---|---|---|---|---|---|---|---|---|---|---|---|---|---|---|
| 1 | 8.21 | 2602900 | 1135811 | 2043295 | 1749663 | 1674462 | 1585609 | 1355802 | 1735617 | 2191187 | 1707559 | 2176556 | 1631738 | 1635171 | 2495830 |
| 2 | 8.21 | 691147 | 285006 | 482299 | 424555 | 384233 | 364592 | 332529 | 425099 | 539733 | 424604 | 518152 | 385313 | 412692 | 605460 |
| 3 | 8.21 | 603956 | 245497 | 417639 | 355878 | 324827 | 277666 | 280839 | 352738 | 461850 | 348183 | 441057 | 318161 | 337446 | 493036 |
| 4 | 13.56 | 1589232 | 772328 | 1662133 | 1165768 | 1191490 | 1024854 | 1058062 | 1254722 | 1747153 | 1294251 | 1415671 | 1352108 | 1181663 | 1888797 |
| 5 | 13.56 | 813838 | 344901 | 638390 | 474985 | 481163 | 434691 | 423256 | 522667 | 729575 | 516737 | 688022 | 533924 | 527308 | 829485 |
| 6 | 13.56 | 660863 | 318965 | 536804 | 430856 | 440272 | 345997 | 364280 | 475079 | 646123 | 462717 | 543303 | 502375 | 447866 | 763599 |
| 7 | 12.07 | 597804 | 505306 | 236376 | 511016 | 378417 | 406026 | 372138 | 436373 | 515898 | 452980 | 420478 | 268442 | 558061 | 530580 |
| 8 | 12.07 | 1291611 | 1094810 | 520779 | 1214566 | 822456 | 843819 | 788668 | 1022282 | 1050848 | 975063 | 1013669 | 565911 | 1283957 | 1249293 |
| 9 | 12.07 | 706081 | 759929 | 309939 | 687667 | 491624 | 520457 | 485558 | 574447 | 548716 | 605390 | 598449 | 338470 | 723065 | 732996 |
| 10 | 17.29 | 385153 | 368340 | 226685 | 407656 | 310474 | 247360 | 323751 | 387635 | 347379 | 241777 | 379235 | 243647 | 403702 | 394797 |
| 11 | 17.29 | 304112 | 310535 | 188282 | 349590 | 226314 | 224923 | 270712 | 327707 | 321914 | 187875 | 328347 | 198322 | 314347 | 315192 |
| 12 | 17.29 | 699754 | 646563 | 420211 | 674373 | 511392 | 437452 | 558446 | 677733 | 602736 | 419494 | 651221 | 423337 | 717561 | 679091 |
| 13 | 10.59 | 341891 | 347557 | 253847 | 418461 | 318267 | 296716 | 261781 | 281010 | 400839 | 300378 | 352239 | 241120 | 347371 | 537144 |
| 14 | 10.59 | 261274 | 278913 | 201016 | 310809 | 250488 | 253221 | 216294 | 223729 | 316939 | 248301 | 268465 | 198992 | 277367 | 435580 |
| 15 | 10.59 | 885464 | 939121 | 671103 | 1035831 | 875375 | 863389 | 813391 | 778143 | 1036135 | 866950 | 857007 | 742315 | 977637 | 1446088 |
| 16 | 9.5 | 250472 | 231109 | 140189 | 281682 | 179053 | 146700 | 177904 | 176637 | 275555 | 195036 | 271337 | 154441 | 217785 | 338233 |
| 17 | 9.5 | 764445 | 747671 | 490987 | 860209 | 590017 | 497323 | 520212 | 574426 | 859168 | 567918 | 797482 | 448122 | 668638 | 1081072 |
| 18 | 9.5 | 403711 | 352493 | 227892 | 453175 | 251970 | 263181 | 241959 | 280867 | 386354 | 275512 | 362660 | 241679 | 308258 | 527817 |
| 19 | 7.55 | 22794 | 42877 | 201618 | 148180 | 25803 | 34431 | 66532 | 78831 | 23453 | 46530 | 26633 | 353334 | 58051 | 0 |
| 20 | 7.55 | 32057 | 51478 | 310930 | 208289 | 37545 | 48019 | 110222 | 114327 | 40798 | 57896 | 33071 | 548761 | 85915 | 0 |
| 21 | 7.55 | 21980 | 21335 | 144042 | 115419 | 17011 | 29434 | 57224 | 64057 | 23170 | 30713 | 23984 | 290918 | 40643 | 0 |
| 22 | 7.55 | 21771 | 30432 | 171063 | 117314 | 23990 | 34970 | 61882 | 74055 | 26827 | 31168 | 25857 | 308266 | 45519 | 0 |
| 23 | 10.96 | 21459 | 41163 | 178441 | 128361 | 26789 | 31210 | 65820 | 67684 | 40275 | 35592 | 28371 | 284750 | 49176 | 0 |
| 24 | 10.96 | 26721 | 24791 | 148910 | 116984 | 19801 | 34184 | 64027 | 62573 | 31666 | 31985 | 29338 | 252856 | 47911 | 0 |
| 25 | 10.97 | 19129 | 42867 | 144327 | 135081 | 19983 | 34148 | 63955 | 68235 | 25278 | 36277 | 19011 | 262190 | 53908 | 0 |
| 26 | 10.97 | 84431 | 149295 | 728764 | 517076 | 86239 | 143900 | 272793 | 264296 | 119628 | 149990 | 107474 | 1161885 | 215385 | 0 |
| 27 | 20.25 | 26516 | 28445 | 95314 | 139753 | 24336 | 20245 | 53755 | 83282 | 28086 | 25551 | 23361 | 258041 | 43989 | 11771 |
| 28 | 20.25 | 33288 | 46900 | 193689 | 242948 | 42220 | 50043 | 106295 | 145502 | 37687 | 49198 | 35696 | 486885 | 84135 | 15498 |
| 29 | 20.24 | 24975 | 37686 | 73554 | 112003 | 20636 | 36909 | 46319 | 65511 | 28916 | 29342 | 25842 | 179672 | 58527 | 14598 |
| 30 | 20.23 | 12603 | 11781 | 39098 | 45394 | 15619 | 9993 | 22964 | 30920 | 12439 | 14732 | 11832 | 113822 | 21066 | 5949 |
| 31 | 13.79 | 0 | 0 | 428755 | 324591 | 0 | 105409 | 141916 | 160279 | 0 | 0 | 0 | 734655 | 119262 | 0 |
| 32 | 13.74 | 0 | 0 | 216751 | 165610 | 0 | 35262 | 134179 | 130839 | 0 | 0 | 0 | 403782 | 157519 | 0 |
| 33 | 13.79 | 0 | 0 | 173261 | 113589 | 0 | 35440 | 59957 | 67094 | 0 | 0 | 0 | 310682 | 38655 | 0 |
| 34 | 13.74 | 0 | 0 | 324871 | 318296 | 0 | 48402 | 179210 | 221336 | 0 | 0 | 0 | 759405 | 210945 | 0 |
| 35 | 18.69 | 12484 | 17815 | 15635 | 24176 | 28003 | 21023 | 24919 | 29824 | 36592 | 21599 | 26365 | 21727 | 32439 | 35384 |
| 36 | 18.68 | 10221 | 25568 | 8955 | 13393 | 19866 | 16092 | 17310 | 18552 | 24709 | 15786 | 15973 | 15705 | 19318 | 26474 |
| 37 | 18.7 | 7938 | 7861 | 6593 | 14178 | 7251 | 12094 | 12662 | 13714 | 16068 | 7151 | 8450 | 7316 | 8752 | 11311 |
| 38 | 16.6 | 6823783 | 7095173 | 4322553 | 7122446 | 7038371 | 6091649 | 5770195 | 7807046 | 8826333 | 5766064 | 7208765 | 6107467 | 8646534 | 10398391 |
| 39 | 16.6 | 13234716 | 14316980 | 8537852 | 14616872 | 14393343 | 12323927 | 11828717 | 15699466 | 17997385 | 12827639 | 13818249 | 12222572 | 18239536 | 21730452 |
| 40 | 16.6 | 4414593 | 4984167 | 3228275 | 4882250 | 4955076 | 4220413 | 3932741 | 5319975 | 5634485 | 4613587 | 4772785 | 4409251 | 6149543 | 7449846 |
| 41 | 16.6 | 17018861 | 17733765 | 10486877 | 17514993 | 18264156 | 15910007 | 14800049 | 20457351 | 22084283 | 16026918 | 16994574 | 15710318 | 21976894 | 26544238 |
| 42 | 13.33 | 20843351 | 24233983 | 14633316 | 21089701 | 22792467 | 18798003 | 18374834 | 24668998 | 24928939 | 22225707 | 22372146 | 20207629 | 27691598 | 30802356 |
| 43 | 13.34 | 17061688 | 20355792 | 12056353 | 16174324 | 18654047 | 15893940 | 14535503 | 19211997 | 20639972 | 17707325 | 17741236 | 16925920 | 22659981 | 26311219 |
| 44 | 13.33 | 19342185 | 23139911 | 13370591 | 19069750 | 19745073 | 17051971 | 16827141 | 23748709 | 24494735 | 18832443 | 20549113 | 19225755 | 25390140 | 29319615 |
| 45 | 13.34 | 5512710 | 7247720 | 4272088 | 5741809 | 6162840 | 5422699 | 5272579 | 6795997 | 7813185 | 6210492 | 6348748 | 6098816 | 8044607 | 9157173 |
| 46 | 14.54 | 265701 | 1168515 | 557899 | 1030702 | 349311 | 1299024 | 1945338 | 2097061 | 2514082 | 1479450 | 54274 | 2084443 | 2317251 | 1079249 |
| 47 | 14.54 | 395416 | 1828630 | 960356 | 1722208 | 628930 | 2111329 | 3186586 | 3495187 | 4254235 | 2438774 | 89752 | 3472589 | 3802123 | 1892483 |
| 48 | 14.54 | 682678 | 3104158 | 1564435 | 2873010 | 976208 | 3572083 | 5244566 | 5377745 | 6843309 | 3848091 | 142623 | 5509593 | 6469904 | 2903631 |
| 49 | 17.03 | 8248555 | 7668553 | 4633613 | 6198627 | 6534736 | 6923653 | 7118401 | 7492835 | 10152365 | 7872583 | 6378013 | 5087023 | 11177637 | 9840259 |
| 50 | 17.03 | 20102696 | 18353666 | 11640756 | 15785641 | 17637157 | 18247283 | 17777482 | 18796824 | 25097722 | 20216262 | 16538603 | 13374262 | 27372458 | 24593515 |
| 51 | 17.03 | 16354432 | 14751562 | 10307458 | 13081573 | 13513838 | 14215725 | 14112799 | 14980515 | 20549684 | 16602607 | 13113796 | 11105624 | 21740608 | 19852094 |
| 52 | 17.03 | 4363285 | 3969736 | 2764420 | 3468815 | 3893693 | 3860568 | 3672677 | 4028991 | 5582634 | 4446618 | 3412893 | 2818500 | 5965451 | 5701247 |
| 53 | 12.15 | 0 | 0 | 0 | 0 | 0 | 85278 | 0 | 0 | 0 | 0 | 0 | 0 | 93929 | 0 |
| 54 | 12.15 | 0 | 0 | 0 | 0 | 0 | 89676 | 0 | 0 | 0 | 0 | 0 | 0 | 81137 | 0 |
| 55 | 12.15 | 0 | 0 | 0 | 0 | 0 | 230756 | 0 | 0 | 0 | 0 | 0 | 0 | 241151 | 0 |
| 56 | 12.15 | 0 | 0 | 0 | 0 | 0 | 101523 | 0 | 0 | 0 | 0 | 0 | 0 | 100430 | 0 |
| 57 | 12.63 | 0 | 0 | 0 | 0 | 0 | 112537 | 0 | 0 | 0 | 0 | 0 | 0 | 109590 | 0 |
| 58 | 12.63 | 0 | 0 | 0 | 0 | 0 | 369953 | 0 | 0 | 0 | 0 | 0 | 0 | 316425 | 0 |
| 59 | **12.63** | 0 | 0 | 0 | 0 | 0 | 392272 | 0 | 0 | 0 | 0 | 0 | 0 | 361642 | 0 |
| 60 | 11.07 | 0 | 0 | 0 | 0 | 0 | 31824 | 0 | 0 | 0 | 0 | 0 | 0 | 32578 | 0 |
| 61 | 11.07 | 0 | 0 | 0 | 0 | 0 | 102500 | 0 | 0 | 0 | 0 | 0 | 0 | 74673 | 0 |
| 62 | 11.08 | 0 | 0 | 0 | 0 | 0 | 50013 | 0 | 0 | 0 | 0 | 0 | 0 | 49681 | 0 |
| 63 | 7.3 | 0 | 0 | 0 | 0 | 0 | 268796 | 0 | 0 | 0 | 0 | 0 | 0 | 317449 | 0 |
| 64 | 7.3 | 0 | 0 | 0 | 0 | 0 | 29511 | 0 | 0 | 0 | 0 | 0 | 0 | 32859 | 0 |
| 65 | 7.3 | 0 | 0 | 0 | 0 | 0 | 60222 | 0 | 0 | 0 | 0 | 0 | 0 | 68361 | 0 |

Les aires des transitions sont sommées par porine et par échantillon afin d'en estimer la quantité. De même, l'ensemble des aires des transitions des protéines de quantification est sommé afin d'estimer la quantité de bactéries. Le ratio de la somme des aires des porines sur la somme des aires des peptides de quantification permet de mesurer le taux d'expression relatif de la porine dans la souche testée. Ce taux d'expression relatif par porine peut également être exprimé en pourcentage du signal moyen par porine sur l'ensemble des souches. Ces résultats sont décrits dans le TABLEAU 18

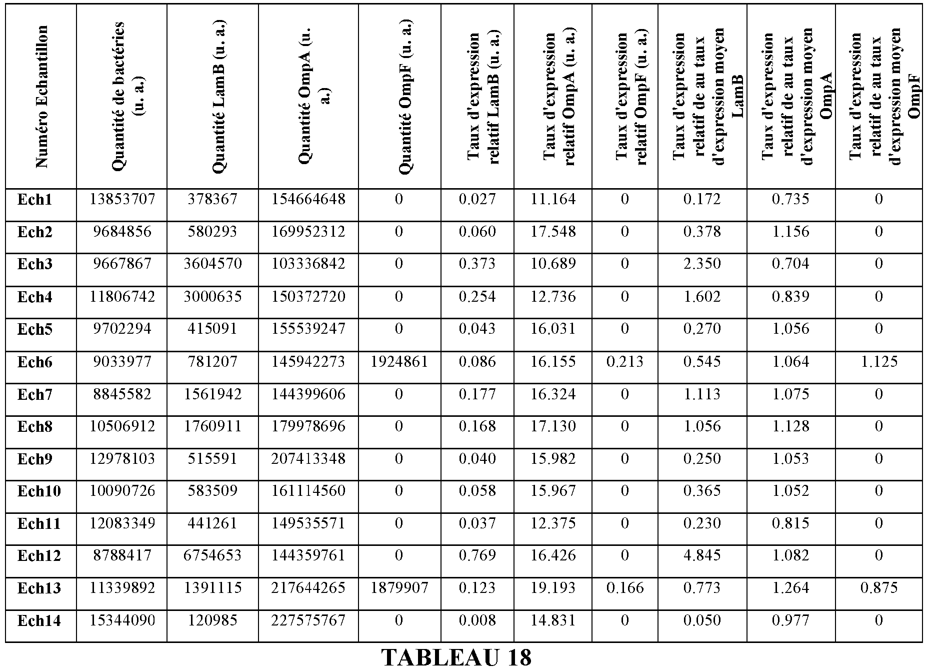

De façon particulièrement avantageuse, les quantités d'OmpA, OmpF, LamB et de bactéries ont été mesurées de façon simultanée à l'aide de la même méthode MRM et nous avons ainsi pu mesurer en une seule analyse le taux d'expression relatif de ces porines. Par exemple, il est possible de constater très facilement que l'échantillon Ech12 à un taux d'expression très élevé de LamB par rapport aux autres échantillons, que les échantillons Ech6 et Ech13 ont un taux d'expression élevé d'OmpF par rapport aux autres échantillons.

### Références bibliographiques

[1] J. Anhalt & C. Fenselau, 1975, Anal. Chem., 47(2) :219-225.
[2] A. Fox et al, ed., 1990, Analytical microbiology methods : chromatography and mass spectrometry, Plenum Press, New York, N.Y.
[3] M. Claydon et al, 1996, Nature Biotech. 14 :1584-1586.
[4] T. Krishnamurthy & P. Ross, 1996, Rapid Com. Mass Spec., 10 :1992-1996.
[5] P. Seng et al. 2009, Clin. Infect. Dis., 49 :543-551.
[6] A. Otto et al. 2012, Curr Opin Microbiol. 15(3):364-72.
[7] P. Dalgaard et al. 1994, Int J Food Microbiol, 23(3-4):391-404.
[8] TJ. Gentry et al. 2006, Microb Ecol., 52(2):159-75.
[9] CY. Cohen et al. 1989, J Clin Microbiol, 27(6): 1250-1256.
[10] R. Everley et al., 2009, J. Microbiol. Methods, 77:152-158.
[11] W.-J. Chen et al., 2008, Anal. Chem., 80 : 9612-9621
[12] D. Lopez-Ferrer et al., 2008, Anal. Chem., 80 :8930-8936
[13] D. Lopez-Ferrer et al., 2005, J. Proteome res., 4(5) : 1569-1574
[14] T. Fortin et al., 2009, Mol. Cell Proteomics, 8(5) : 1006-1015.
[15] H. Keshishian et al., 2007, Mol. Cell Proteomics, 2212-2229.
[16] J. Stal-Zeng et al., 2007, Mol. Cell Proteomics, 1809-1817.
[17] Gaskell, Electrospray: principles and practise, 1997, J. Mass Spectrom., 32, 677-688).
[18] V. Fusaro et al., 2009, Nature Biotech. 27, 190-198.
[19] J. Mead et al., 15 nov 2008, Mol. Cell Proteomics, E-pub.
[20] F. Desiere et al., 2006, Nucleic Acids Res., 34(database issue) : D655-8).
[21] L. Anderson & C. Hunter, 2006, Mol. Cell Proteomics, 573-588).
[22] B. Han & R. Higgs, 2008, Brief Funct Genomic Proteomic.,7(5):340-54).
[23] K.-Y. Wang et al., 2008, Anal Chem, 80(16) 6159-6167).
[24] J. Bundy & C. Fenselau, 1999, Anal. Chem. 71 : 1460-1463.
[25] JD. Venable et al. 2004, Nat Methods. 1(1):39-45.
[26] HL. Röst et al.2014 Nat Biotechnol. 32(3):219-23.
[27] RS. Plumb et al, 2006, Rapid Commun Mass Spectrom. 20(13):1989-94.
[28] K-C Ho et al., 2004, Anal. Chem. 76 : 7162-7268.
[29] Y.S. Lin et al., 2005, Anal. Chem., 77 : 1753-1760.
[30] S. Vaidyanathan et al., 2001, Anal. Chem., 73 :4134-4144.
[31] P. Seng et al., 2009, Clin. Infect. Dis., 49 :543-551.
[32] Manes N. et al., 2007, Mol. & Cell. Proteomics, 6(4): 717-727.
[33] R. Nandakumar et al., 2009, Oral Microbiology Immunology, 24 :347-352.
[34] L. Hernychova et al., 2008, Anal. Chem., 80 :7097-7104.
[35] J.-M. Pratt et al., 2006, Nat. Protoc., 1: 1029-1043.
[36] V. Brun et al., 2007, Mol. Cell Proteomics, 2139-2149.
[37] M. Dupont et al., 2007, Antimicrobial agents and chemotherapy, 51 : 3190-3198.

## Revendications

1. Procédé de quantification d'au moins un groupe de microorganismes par au moins une analyse en spectrométrie de masse comprenant au moins une étape de séparation et de fragmentation, ledit procédé comprenant par ailleurs une étape consistant à mesurer la quantité d'au moins un peptide représentatif dudit groupe de microorganismes dont la quantité produite ou exprimée ne varie pas en fonction de la phase de croissance dans laquelle se trouve ledit groupe de microorganismes, ni en fonction du type de culture ayant subi ledit groupe de microorganismes, ni en fonction des souches utilisées faisant partie d'un seul et même groupe de microorganismes, ledit au moins un peptide représentatif étant obtenu après l'étape de séparation et de fragmentation et jouant le rôle de marqueur(s) de quantification, la quantité dudit ou desdits marqueur(s) de quantification étant directement corrélable à la quantité du groupe de microorganismes,
ledit procédé étant **caractérisé en ce que** :
- le groupe de microorganismes est constitué par l'espèce *Pseudomonas aeruginosa* et ledit peptide est de séquence SEQ ID NO :6, et/ou
- le groupe de microorganismes est constitué par l'espèce *Escherichia coli* et ledit peptide est de séquence SEQ ID NO :2, et/ou
- le groupe de microorganismes est constitué par l'espèce *Staphylococcus aureus* et ledit peptide est de séquence SEQ ID NO :4 et, et/ou
- le groupe de microorganismes est constitué par la famille des Entérobactéries et ledit peptide est de séquence SEQ ID NO :8.

2. Procédé selon la revendication 1, comprenant une étape consistant à réaliser l'identification du au moins un groupe de microorganismes simultanément à la quantification dudit au moins un groupe de microorganismes.

3. Procédé selon la revendication 1 ou 2, dans lequel la spectrométrie de masse est de type PRM, SRM, MRM, MS², MRM³, DDA (data dependent acquisition) ou DIA (data independent acquisition).

4. Procédé de mesure du taux d'expression d'au moins un peptide et/ou d'au moins une protéine d'intérêt d'un groupe de microorganismes, par au moins une analyse en spectrométrie de masse comprenant au moins une étape de séparation et de fragmentation, ledit procédé comprenant les étapes suivantes :
a) Mesurer la quantité d'au moins un peptide d'intérêt et/ou d'au moins une protéine d'intérêt,
b) mesurer la quantité d'au moins un peptide représentatif dudit groupe de microorganismes, dont la quantité produite ou exprimée ne varie pas en fonction de la phase de croissance dans laquelle se trouve ledit groupe de microorganismes, ni en fonction du type de culture ayant subi ledit groupe de microorganismes, ni en fonction des souches utilisées faisant partie d'un seul et même groupe de microorganismes, jouant le rôle de marqueur(s) de quantification, la quantité dudit ou desdits marqueur(s) de quantification étant directement corrélable à la quantité du au moins un groupe de microorganismes,
c) déduire le taux d'expression du au moins un peptide et/ou de la au moins une protéine d'intérêt dudit groupe de microorganismes,
le au moins un peptide d'intérêt et/ou la au moins une protéine d'intérêt, ainsi que le au moins un peptide représentatif ou la au moins une protéine représentative dudit groupe de microorganismes étant obtenus après la au moins une étape de séparation et de fragmentation,
ledit procédé étant **caractérisé en ce que** :
- le groupe de microorganismes est constitué par le genre *Enterococcus* et ledit peptide représentatif est choisi parmi les peptides de séquences SEQ ID NOs : 10, 12, et 14, et/ou
- le groupe de microorganismes est constitué par l' espèce *Klebsiella pneumoniae* et ledit peptide représentatif est choisi parmi les peptides de séquences SEQ ID NOs : 16, 18, et 20.

5. Procédé selon la revendication 4, dans lequel l'étape c) consiste à comparer la quantité mesurée du au moins un peptide d'intérêt et/ou de la au moins une protéine d'intérêt à la quantité du au moins un peptide représentatif ou de la au moins une protéine représentative dudit groupe de microorganismes jouant le rôle de marqueur(s) de quantification, de sorte qu'il est ainsi possible de déduire un taux d'expression relatif par rapport à la quantité dudit ou desdits marqueur(s) de quantification.

6. Procédé selon la revendication 4 ou 5, comportant une étape complémentaire bi) consistant à déterminer la quantité du au moins un groupe de microorganismes à partir de la quantité dudit ou desdits marqueur(s) de quantification, obtenue à l'étape b).

7. Procédé selon l'une des revendications 4 à 6, dans lequel la spectrométrie de masse est de type PRM, SRM, MRM, MRM³, DDA (data dependent acquisition) ou DIA (data independent acquisition).

## Patentansprüche

1. Verfahren zur Quantifizierung von mindestens einer Mikroorganismusgruppe durch mindestens eine Massenspektrometrie-Analyse, umfassend mindestens einen Schritt der Trennung und Fragmentierung, wobei das Verfahren unter anderem einen Schritt bestehend aus dem Messen der Menge mindestens eines repräsentativen Peptids der Mikroorganismusgruppe umfasst, dessen produzierte oder exprimierte Menge weder abhängig von der Wachstumsphase, in der sich die Mikroorganismusgruppe befindet, noch abhängig vom Kulturtyp, dem die Mikroorganismusgruppe unterzogen wird, noch abhängig von den verwendeten Stämmen, die Teil ein und derselben Mikroorganismusgruppe sind, variiert, wobei das mindestens eine repräsentative Peptid nach dem Schritt der Trennung und Fragmentierung erhalten wird und die Rolle von Quantifizierungsmarker(n) erfüllt, wobei die Menge des bzw. der Quantifizierungsmarker(s) direkt mit der Menge der Mikroorganismusgruppe korrelierbar ist, wobei das Verfahren **dadurch gekennzeichnet ist, dass**:
- die Mikroorganismusgruppe aus der Spezies *Pseudomonas aeruginosa* besteht und das Peptid die Sequenz SEQ ID NO:6 aufweist, und/oder
- die Mikroorganismusgruppe aus der Spezies *Escherichia coli* besteht und das Peptid die Sequenz SEQ ID NO:2 aufweist, und/oder
- die Mikroorganismusgruppe aus der Spezies *Staphylococcus aureus* besteht und das Peptid die Sequenz SEQ ID NO:4 aufweist, und/oder
- die Mikroorganismusgruppe aus der Familie der Enterobakterien besteht und das Peptid die Sequenz SEQ ID NO:8 aufweist.

2. Verfahren gemäß Anspruch 1, umfassend einen Schritt bestehend aus dem Durchführen der Identifizierung der mindestens einen Mikroorganismusgruppe gleichzeitig mit der Quantifizierung der mindestens einen Mikroorganismusgruppe.

3. Verfahren gemäß Anspruch 1 oder 2, wobei es sich beim Typ der Massenspektrometrie um PRM, SRM, MRM, MS², MRM³, DDA (data-dependent acquisition) oder DIA (data-independent acquisition) handelt.

4. Verfahren zur Messung des Expressionsniveaus mindestens eines Peptids und/oder mindestens eines Proteins von Interesse einer Mikroorganismusgruppe durch mindestens eine Massenspektrometrie-Analyse, umfassend mindestens einen Schritt der Trennung und Fragmentierung, wobei das Verfahren die folgenden Schritte umfasst:
a) Messen der Menge mindestens eines Peptids von Interesse und/oder mindestens eines Proteins von Interesse,
b) Messen der Menge mindestens eines repräsentativen Peptids der Mikroorganismusgruppe, dessen produzierte oder exprimierte Menge weder abhängig von der Wachstumsphase, in der sich die Mikroorganismusgruppe befindet, noch abhängig vom Kulturtyp, dem die Mikroorganismusgruppe unterzogen wird, noch abhängig von den verwendeten Stämmen, die Teil ein und derselben Mikroorganismusgruppe sind, variiert, und das die Rolle von Quantifizierungsmarker(n) erfüllt, wobei die Menge des bzw. der Quantifizierungsmarker(s) direkt mit der Menge der mindestens einen Mikroorganismusgruppe korrelierbar ist,
c) Ableiten des Expressionsniveaus des mindestens einen Peptids und/oder des mindestens einen Proteins von Interesse der Mikroorganismusgruppe,
wobei das mindestens eine Peptid von Interesse und/oder das mindestens eine Protein von Interesse sowie das mindestens eine repräsentative Peptid oder das mindestens eine repräsentative Protein der Mikroorganismusgruppe nach dem mindestens einen Schritt der Trennung und Fragmentierung erhalten werden,
wobei das Verfahren **dadurch gekennzeichnet ist, dass**:
- die Mikroorganismusgruppe aus der Gattung *Enterococcus* besteht und das repräsentative Peptid ausgewählt ist aus den Peptiden mit den Sequenzen SEQ ID NOs: 10, 12 und 14, und/oder
- die Mikroorganismusgruppe aus der Spezies *Klebsiella pneumoniae* besteht und das repräsentative Peptid ausgewählt ist aus den Peptiden mit den Sequenzen SEQ ID NOs: 16, 18 und 20.

5. Verfahren gemäß Anspruch 4, wobei der Schritt c) aus dem Vergleichen der gemessenen Menge des mindestens einen Peptids von Interesse und/oder des mindestens einen Proteins von Interesse mit der Menge des mindestens einen repräsentativen Peptids oder des mindestens einen repräsentativen Proteins der Mikroorganismusgruppe, das die Rolle von Quantifizierungsmarker(n) erfüllt, besteht, wodurch es ermöglicht wird, ein relatives Expressionsniveau in Bezug auf die Menge des bzw. der Quantifizierungsmarker(s) abzuleiten.

6. Verfahren gemäß Anspruch 4 oder 5, das einen zusätzlichen Schritt b₁) umfasst, bestehend aus dem Bestimmen der Menge der mindestens einen Mikroorganismusgruppe basierend auf der Menge des bzw. der Quantifizierungsmarker(s), die im Schritt b) erhalten wurde.

7. Verfahren gemäß einem der Ansprüche 4 bis 6, wobei es sich beim Typ der Massenspektrometrie um PRM, SRM, MRM, MRM³, DDA (data-dependent acquisition) oder DIA (data-independent acquisition) handelt.

## Claims

1. A method for quantifying at least one microorganism group via at least one mass spectrometry analysis comprising at least one separation and fragmentation step, the method comprising, moreover, a step consisting in measuring the amount of at least one peptide representative of the microorganism group which the amount produced or expressed does not vary according to the growth phase in which said microorganism group is situated, or as a function of the type of culture having been subjected to said microorganism group, or as a function of the strains used that are part of a single one and the same microorganism group, the at least one representative peptide being obtained after the separation and fragmentation step and serving as quantification marker(s), the amount of the quantification marker(s) being directly correlatable to the amount of the microorganism group,
and wherein:
- the microorganism group consists of *Pseudomonas aeruginosa* species and said representative peptide is sequence SEQ ID NO: 6, and/or
- the microorganism group consists of *Escherichia coli* species and said representative peptide is sequence SEQ ID NO: 2, and/or
- the microorganism group consists of *Staphilococcus aureus* species and said representative peptide is sequence SEQ ID NO: 4, and/or
- the microorganism group consists of the Enterobacteria family and said representative peptide is sequence SEQ ID NO: 8.

2. The method as claimed in claim 1, comprising a step consisting in carrying out the identification of the at least one microorganism group simultaneously with the quantification of the at least one microorganism group.

3. The method as claimed in claim 1, wherein the mass spectrometry is of PRM, SRM, MRM, MS², MRM³, DDA (data dependent acquisition) or DIA (data independent acquisition) type.

4. A method for measuring the level of expression of at least one peptide and/or of at least one protein of interest of a microorganism group, by means of at least one mass spectrometry analysis comprising at least one separation and fragmentation step, the method comprising the following steps:
a) measuring the amount of at least one peptide of interest and/or of at least one protein of interest,
b) measuring the amount of at least one peptide representative of the microorganism group, which the amount produced or expressed does not vary according to the growth phase in which said microorganism group is situated, or as a function of the type of culture having been subjected to said microorganism group, or as a function of the strains used that are part of a single one and the same microorganism group, serving as quantification marker(s), the amount of the quantification marker(s) being directly correlatable to the amount of the at least one microorganism group,
c) deducing the level of expression of the at least one peptide and/or of the at least one protein of interest of the microorganism group,
the at least one peptide of interest and/or the at least one protein of interest, and also the at least one peptide representative or the at least one protein representative of the microorganism group, being obtained after the at least one separation and fragmentation step,
and wherein:
- the microorganism group consists of *Enterococcus* genus and said representative peptide is chosen among peptide sequences SEQ ID NOs: 10, 12 and 14, and/or
- the microorganism group consists of *Klebsiella pneumoniae* species and said representative peptide is chosen among peptide sequences SEQ ID NO: 16, 18 and 20.

5. The method as claimed in claim 4, wherein step c) consists in comparing the measured amount of the at least one peptide of interest and/or of the at least one protein of interest with the amount of the at least one peptide representative or of the at least one protein representative of the microorganism group serving as quantification marker(s), such that it is thus possible to deduce a relative expression level with respect to the amount of the quantification marker(s).

6. The method as claimed in claim 4 or 5, comprising an additional step bi) consisting in determining the amount of the at least one microorganism group on the basis of the amount of the quantification marker(s) obtained in step b).

7. The method as claimed in any one of claims 4 to 6, wherein the mass spectrometry is of PRM, SRM, MRM, MRM³, DDA (data dependent acquisition) or DIA (data independent acquisition) type.
